(19) Europäisches Patentamt
European Patent Office
Office européen des brevets

(11) **EP 4 303 462 A1**

(12) **EUROPEAN PATENT APPLICATION**
published in accordance with Art. 153(4) EPC

(43) Date of publication:
**10.01.2024 Bulletin 2024/02**

(21) Application number: **22763137.1**

(22) Date of filing: **25.02.2022**

(51) International Patent Classification (IPC):
*F16D 43/202* (2006.01)   *A61F 2/62* (2006.01)
*F16H 21/10* (2006.01)   *F16H 25/20* (2006.01)
*F16H 35/00* (2006.01)   *F16H 37/12* (2006.01)

(52) Cooperative Patent Classification (CPC):
**A61F 2/60; F16D 43/202; F16H 21/10; F16H 25/20;
F16H 35/00; F16H 37/12**

(86) International application number:
**PCT/JP2022/007967**

(87) International publication number:
**WO 2022/186081 (09.09.2022 Gazette 2022/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.03.2021 JP 2021032992**

(71) Applicant: **HONDA MOTOR CO., LTD.**
**Minato-ku**
**Tokyo 107-8556 (JP)**

(72) Inventor: **TAKAMI, Eika**
**Wako-shi, Saitama 351-0193 (JP)**

(74) Representative: **Weickmann & Weickmann
PartmbB
Postfach 860 820
81635 München (DE)**

(54) **JOINT DEVICE**

(57) A motorized artificial leg (1) comprises a below-knee member (10), an above-knee member (20), a knee joint mechanism (30) that links the below-knee member (10) and the above-knee member (20) such that the angle formed therebetween can be changed, and a lengthening and shortening device (40) that can change the angle formed between the below-knee member (10) and the above-knee member (20) by lengthening and shortening. The lengthening and shortening device (40) comprises a motor (M) and a transmission (T) that transmits motive force from the motor (M). The transmission (T) comprises a first transmission mechanism (T1) that transmits motive force from the motor (M) at a first gear ratio and a second transmission mechanism (T2) that transmits motive force from the motor (M) at a second gear ratio that is different from the first gear ratio.

*FIG. 3*

## Description

### TECHNICAL FIELD

The present invention relates to a joint device.

### BACKGROUND ART

In the related art, as a joint device used for a coupling portion coupling two members, there is a joint device including an expansion and contraction device capable of changing an angle formed by the two members. Such a joint device includes, for example, a prosthetic leg used for a knee joint. Patent Literature 1 discloses that a sensor for detecting contraction motion of a muscle at a cut-off end portion of an amputated leg is provided in a thigh socket of a prosthetic leg attached to the cut-off end portion of the amputated leg, and a throttling condition of a variable valve of a hydraulic cylinder for adjusting resistance of bending and stretching of a knee joint portion is controlled based on detection information from the sensor.

### CITATION LIST

### PATENT LITERATURE

Patent Literature 1: JP-A-H11-19105

### SUMMARY OF INVENTION

### TECHNICAL PROBLEM

However, in the prosthetic leg disclosed in Patent Literature 1, although resistance of bending and stretching can be generated, power of bending and stretching cannot be generated. In particular, in order to smoothly ascend steps, it is necessary to stretch the knee joint in a state where a load is applied.
The present invention provides a joint device capable of stretching and bending a coupling portion by power of a power source.

### SOLUTION TO PROBLEM

A first invention provides a joint device including:

a first member;
a second member;
a coupling portion which couples the first member and the second member so that an angle formed by the first member and the second member is changeable; and
an expansion and contraction device configured to change the angle formed by the first member and the second member by expanding and contracting, in which:

the expansion and contraction device includes:

a power source; and
a power transmission portion configured to transmit power of the power source;

the power transmission portion includes:

a first power transmission path configured to transmit the power at a first transmission gear ratio; and
a second power transmission path configured to transmit the power at a second transmission gear ratio different from the first transmission gear ratio;

the expansion and contraction device includes:

a first connection and disconnection mechanism configured to switch disconnection and connection of power in the first power transmission path; and
a second connection and disconnection mechanism configured to switch disconnection and connection of power in the second power transmission path;

the joint device further includes:

a control unit configured to control the power source, the first connection and disconnection mechanism, and the second connection and disconnection mechanism; and
an applied weight acquisition unit which acquires an applied weight applied to the joint device, and
the control unit controls at least one of the first connection and disconnection mechanism and the second connection and disconnection mechanism based on the applied weight acquired by the applied weight acquisition unit.

A second invention provides a joint device including:

a first member;
a second member;
a coupling portion which couples the first member and the second member so that an angle formed by the first member and the second member is changeable; and
an expansion and contraction device configured to change the angle formed by the first member and the second member by expanding and contracting, in which:

the expansion and contraction device includes:

a power source; and
a power transmission portion configured to transmit power of the power source;

the second member is configured to be attachable to the first portion of a wearing subject of the joint device out of the first portion and a second portion which pivots relative to the first portion;
the joint device further includes:

a control unit configured to control the power source; and
a pivoting force acquisition unit configured to acquire a pivoting force of the first portion relative to the second portion; and
the control unit is configured to control an amount of power generated from the power source based on the pivoting force acquired by the pivoting force acquisition unit.

A third invention provides a joint device including:

a first member;
a second member;
a coupling portion which couples the first member and the second member so that an angle formed by the first member and the second member is changeable; and
an expansion and contraction device configured to change the angle formed by the first member and the second member by expanding and contracting, in which:

the expansion and contraction device includes:

a power source; and
a power transmission portion configured to transmit power of the power source;

the power transmission portion includes:

a first power transmission path configured to transmit the power at a first transmission gear ratio; and
a second power transmission path configured to transmit the power at a second transmission gear ratio different from the first transmission gear ratio;

the expansion and contraction device includes:

a first connection and disconnection mechanism configured to switch disconnection and connection of power in the first power transmission path; and
a second connection and disconnection mechanism configured to switch disconnection and connection of power in the second power transmission path;

the joint device further includes:

a control unit configured to control the power source, the first connection and disconnection mechanism, and the second connection and disconnection mechanism; and
a traveling direction acquisition unit configured to acquire a traveling direction of a wearing subject of the joint device; and
the control unit is configured to control at least one of the first connection and disconnection mechanism and the second connection and disconnection mechanism based on the traveling direction acquired by the traveling direction acquisition unit.

ADVANTAGEOUS EFFECTS OF INVENTION

According to the present invention, the coupling portion can be stretched and bent via the power transmission portion which transmits power of the power source.

BRIEF DESCRIPTION OF DRAWINGS

[FIG. 1] FIG. 1 is a perspective view of an electric prosthetic leg according to a first embodiment of the present invention as viewed obliquely from a front.
[FIG. 2] FIG. 2 is a perspective view of the electric prosthetic leg in FIG. 1 as viewed obliquely from a rear.
[FIG. 3] FIG. 3 is a view illustrating an internal structure of the electric prosthetic leg in FIG. 1.
[FIG. 4] FIG. 4 is an enlarged view of a power transmission portion of the electric prosthetic leg in FIG. 1.
[FIG. 5A] FIG. 5A is a view illustrating power transmission of the power transmission portion when the power transmission portion in FIG. 4 is in a first gear shift state.
[FIG. 5B] FIG. 5B is a view illustrating power transmission of the power transmission portion when the power transmission portion in FIG. 4 is in a second gear shift state.
[FIG. 5C] FIG. 5C is a view illustrating power transmission of the power transmission portion when the power transmission portion in FIG. 4 is in a free state.
[FIG. 6] FIG. 6 is a view illustrating stretch of a knee joint mechanism from a bent state when ascending steps.
[FIG. 7] FIG. 7 is a view illustrating bending of the knee joint mechanism from a stretched state when ascending steps.

[FIG. 8] FIG. 8 is a perspective view of the power transmission portion of the electric prosthetic leg according to a second embodiment of the present invention.

[FIG. 9] FIG. 9 is a cross-sectional view of the power transmission portion in FIG. 8.

[FIG. 10] FIG. 10 is a functional block diagram of a control system in a step ascending control according to a first example.

[FIG. 11] FIG. 11 is a diagram illustrating a sensor and the like of the electric prosthetic leg necessary for the step ascending control according to the first example.

[FIG. 12] FIG. 12 is a diagram illustrating a flow when the electric prosthetic leg is on a standing leg side in the step ascending control according to the first example.

[FIG. 13] FIG. 13 is a diagram illustrating a flow when the electric prosthetic leg is on an idling leg side in the step ascending control according to the first example.

[FIG. 14] FIG. 14 is a graph illustrating an applied weight and a hip joint torque at the time of ascending steps.

[FIG. 15] FIG. 15 is a functional block diagram of the control system in the step ascending control according to a second example.

[FIG. 16] FIG. 16 is a diagram illustrating a sensor and the like of the electric prosthetic leg necessary for the step ascending control according to the second example.

[FIG. 17] FIG. 17 is a diagram illustrating a flow when the electric prosthetic leg is on the standing leg side in the step ascending control according to the second example.

[FIG. 18] FIG. 18 is a graph illustrating a relationship between motor control and each of a knee joint torque and a knee angle in the step ascending control according to the second example.

[FIG. 19] FIG. 19 is a graph illustrating an applied weight and a hip joint torque at the time of ascending steps.

DESCRIPTION OF EMBODIMENTS

Hereinafter, each embodiment of an electric prosthetic leg as an example of a joint device of the present invention and step ascending control when ascending steps by using this electric prosthetic leg will be described with reference to the drawings. In the following description, a front-rear direction, a left-right direction, and an upper-lower direction are defined with reference to a user of the electric prosthetic leg. In the drawings, a front side of the electric prosthetic leg is denoted by Fr, a rear side is denoted by Rr, a left side is denoted by L, a right side is denoted by R, an upper side is denoted by U, and a lower side is denoted by D.

<First Embodiment> [Electric Prosthetic Leg]

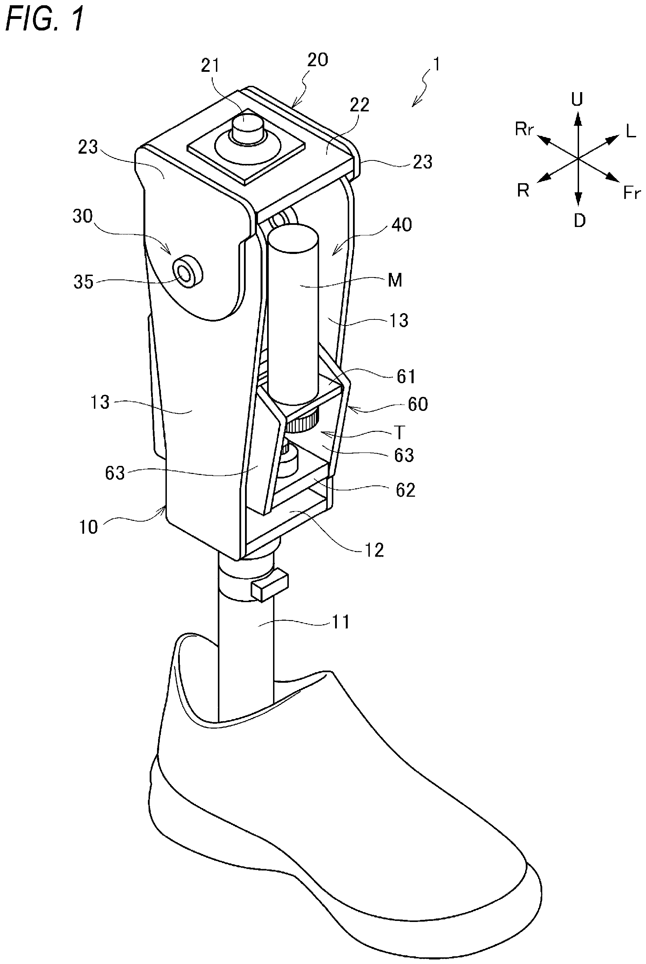

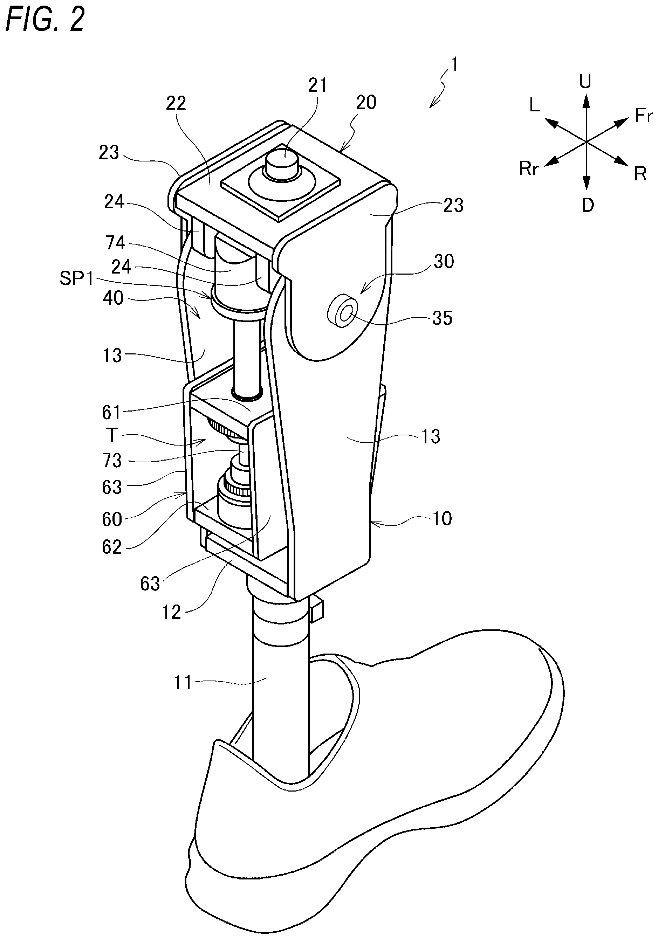

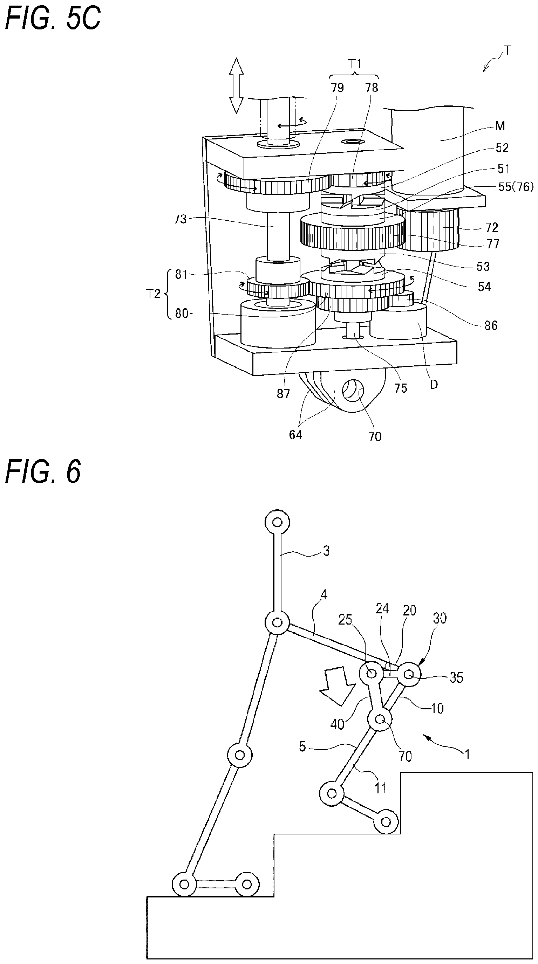

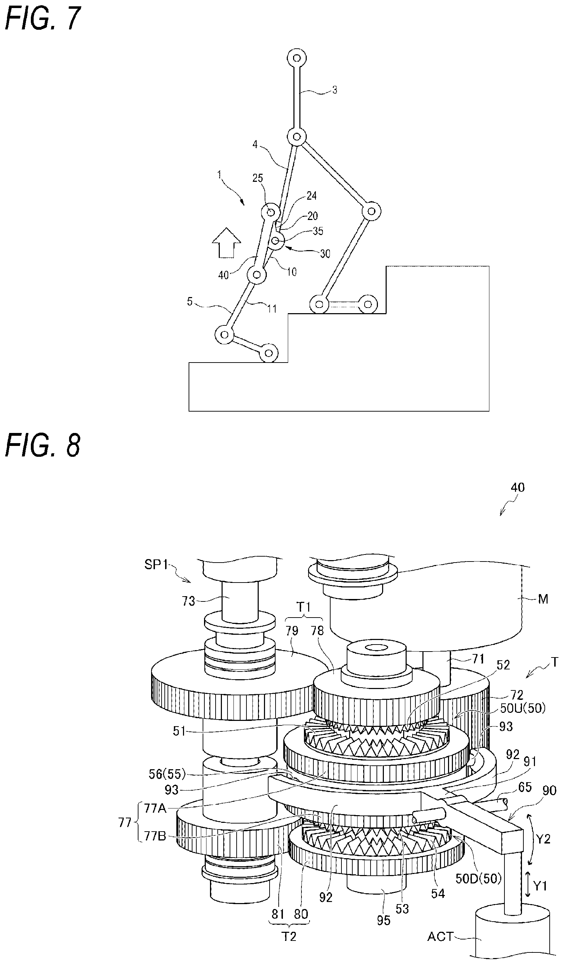

An electric prosthetic leg 1 of the present embodiment is a prosthetic leg for a person without a knee. As illustrated in FIGS. 6 and 7, the electric prosthetic leg 1 is attached to one thigh 4 which pivots relative to an upper body 3 and functions as a lower leg 5. As illustrated in FIGS. 1 to 3, the electric prosthetic leg 1 includes a lower-knee member 10 located on lower side of a knee, an upper-knee member 20 located on upper side of the knee, a knee joint mechanism 30 which couples the lower-knee member 10 and the upper-knee member 20 so that an angle formed by the lower-knee member 10 and the upper-knee member 20 can be changed, and an expansion and contraction device 40 which can change the angle formed by the lower-knee member 10 and the upper-knee member 20 by expanding and contracting.

The upper-knee member 20 includes an upper wall portion 22 provided with an adapter 21 connected to a socket (not illustrated), and a pair of upper side wall portions 23 extending downward from both left and right ends of the upper wall portion 22, and the upper-knee member 20 has a substantially U-shape with a lower opening when viewed from the front-rear direction.

The lower-knee member 10 includes a lower wall portion 12 provided with a leg portion 11, and a pair of lower side wall portions 13 extending upward from both left and right ends of the lower wall portion 12, and has a substantially U-shape with an upper opening when viewed in the front-rear direction.

The pair of lower side wall portions 13 of the lower-knee member 10 are connected between the pair of upper side wall portions 23 of the upper-knee member 20 so as to be rotatable about a pivoting portion 35. By this mechanism, the knee joint mechanism 30 is configured to couple the lower-knee member 10 and the upper-knee member 20 so that the angle formed by the lower-knee member 10 and the upper-knee member 20 can be changed.

The expansion and contraction device 40 capable of changing the angle formed by the lower-knee member 10 and the upper-knee member 20 is provided in a space formed between the upper-knee member 20 and the lower-knee member 10.

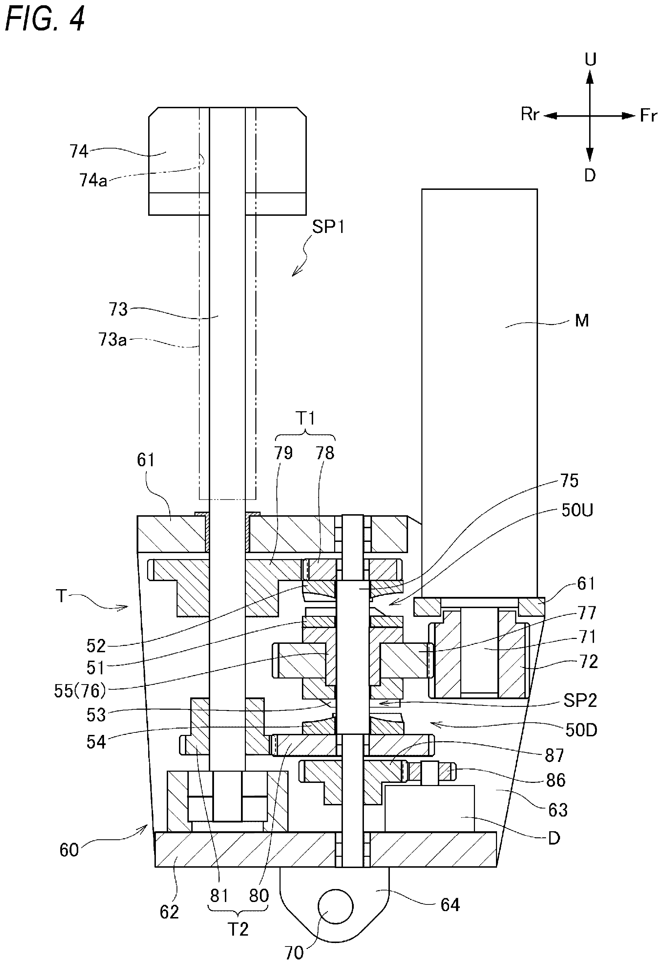

Referring also to FIG. 4, the expansion and contraction device 40 includes a motor M which outputs rotational power, a transmission T which transmits power of the motor M, a first spindle unit SP1 which is connected to the transmission T in a power transmittable manner and converts the rotational power output from the transmission T into translational motion, an connection and disconnection mechanism 50 which is interposed between the motor M and each of a first transmission mechanism T1 and a second transmission mechanism T2 of the transmission T to be described later, and a second spindle unit SP2 which converts the rotational power output from the motor M into translational motion of an operator 55 of the connection and disconnection mechanism 50.

The transmission T includes a transmission case 60 having a rectangular shape when viewed from the front-rear direction and having a top plate portion 61, a bottom plate portion 62, and a pair of side plate portions 63 connecting left and right ends of the top plate portion 61 and the bottom plate portion 62. On the transmission case 60, a pair of pivoting blades 64 extending downward from the bottom plate portion 62 is supported by a pivoting support wall 14 extending upward from a bottom wall portion 12 of the lower-knee member 10 so as to be swingable and immovable about a lower swing portion 70.

The motor M is disposed forward and upward of the top plate portion 61 of the transmission case 60 such that an output shaft 71 protrudes into the transmission case 60 through the top plate portion 61. The first spindle unit SP1 is disposed on an opposite side of the motor M across the connection and disconnection mechanism 50 in the front-rear direction. In other words, the motor M is disposed forward of the connection and disconnection mechanism 50 in the front-rear direction, and the first spindle unit SP1 is disposed rearward of the connection and disconnection mechanism 50 in the front-rear direction. Accordingly, even when the motor M is mounted on the electric prosthetic leg 1, it is possible to prevent an increase in a size of the electric prosthetic leg 1 in a width direction while maintaining a balance in the front-rear direction.

The second spindle unit SP2 which converts the rotational power output from the motor M into translational motion of the operator 55 of the connection and disconnection mechanism 50 is disposed between the motor M and the first spindle unit SP1 in the front-rear direction. The output shaft 71 of the motor M, a first spindle 73 of the first spindle unit SP1, and a second spindle 75 of the second spindle unit SP2 are disposed parallel to each other, and disposed to face the upper-lower direction in a state where the knee joint mechanism 30 is completely stretched.

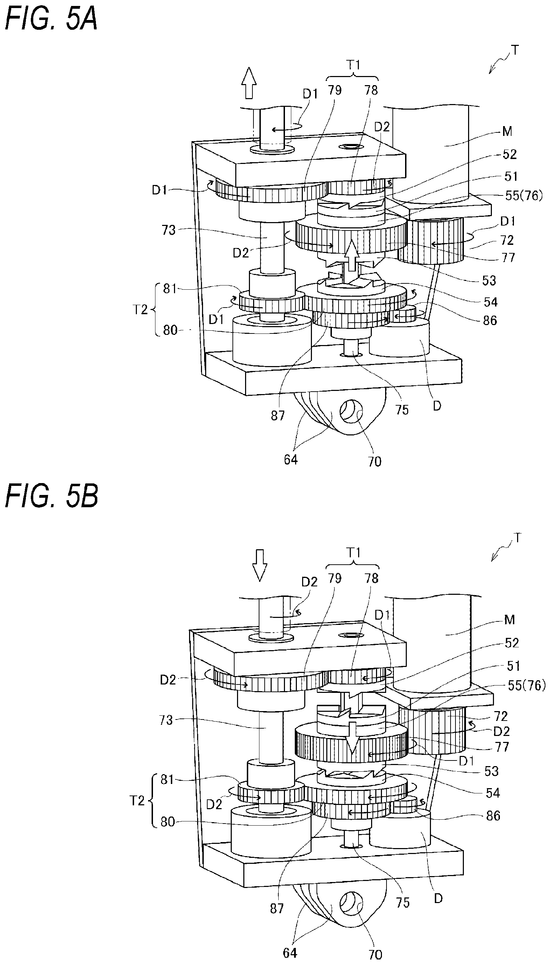

The first spindle unit SP1 includes the first spindle 73 formed with a male thread 73a and a sleeve 74 formed with a female thread 74a. The sleeve 74 performs translational motion along an axis of the first spindle 73 by rotation of the first spindle 73. In the present embodiment, the first spindle 73 performs rotational motion by receiving rotational power of the motor M transmitted by the transmission T. On the other hand, the sleeve 74 is attached to a pair of inner side wall portions 24 extending downward from the upper wall portion 22 of the upper-knee member 20 so as to be swingable and immovable about an upper swing portion 25. Therefore, when the first spindle 73 rotates to one side (in direction of arrow D1 in FIG. 5A) by receiving the rotational power of the motor M transmitted by the transmission T, the sleeve 74 performs translational motion to be separated from the transmission T, and when the first spindle 73 is rotated to the other side (in direction of arrow D2 in FIG. 5B), the sleeve 74 performs translational motion to approach the transmission T.

That is, a distance between the sleeve 74 and the transmission T increases and decreases in accordance with a rotation direction of the first spindle 73. Since the sleeve 74 is immovably attached to the upper-knee member 20 as described above, the distance between the sleeve 74 and the transmission T increases and decreases in accordance with the rotation direction of the first spindle 73. Therefore, the lower-knee member 10 to which the transmission T is attached and the upper-knee member 20 to which the sleeve 74 is attached rotate about the pivoting portion 35. Accordingly, the angle formed by the upper-knee member 20 and the lower-knee member 10 changes.

As illustrated in FIG. 4, the transmission T includes an output gear 72 provided on the output shaft 71 of the motor M, an input gear 77 provided at a substantially central portion of the second spindle 75 of the second spindle unit SP2 and meshing with the output gear 72, the first transmission mechanism T1, and the second transmission mechanism T2.

The first transmission mechanism T1 includes a first drive gear 78 immovably disposed on an upper side of the second spindle 75 of the second spindle unit SP2, and a first driven gear 79 disposed on the first spindle 73 of the first spindle unit SP1 so as to rotate integrally with the first spindle 73 and meshing with the first drive gear 78.

The second transmission mechanism T2 includes a second drive gear 80 immovably disposed on a lower side of the second spindle 75 of the second spindle unit SP2, and a second driven gear 81 disposed on the first spindle 73 of the first spindle unit SP1 so as to rotate integrally with the first spindle 73 and meshing with the second drive gear 80.

The first transmission mechanism T1 serves as a first power transmission path and transmits power of the motor M at a first transmission gear ratio. The second transmission mechanism T2 serves as a second power transmission path and transmits the power of the motor M at a second transmission gear ratio different from the first transmission gear ratio. By providing the two power transmission paths having different transmission gear ratios, it is possible to switch an operation speed and generated power of stretching and bending of the knee joint mechanism 30. As long as the first transmission gear ratio and the second transmission gear ratio are different from each other, one of the first transmission mechanism T1 and the second transmission mechanism T2 may be a speed reduction mechanism and the other may be a speed acceleration mechanism, one of the first transmission mechanism T1 and the second transmission mechanism T2 may be a constant speed mechanism and the other may be a speed reduction mechanism or a speed acceleration mechanism, both of the first transmission mechanism T1 and the second transmission mechanism T2 may be speed reduction mechanisms, and both of the first transmission mechanism T1 and the second transmission mechanism T2 may be speed acceleration mechanisms.

It is preferable that, when the first transmission gear ratio is a ratio of a post-shift rotation speed, which is a rotation speed on an opposite side of the motor M (first spindle unit SP1 side) in the first transmission mechanism T1, to a pre-shift rotation speed, which is a rotation speed on a motor M side in the first transmission mechanism T1, and the second transmission gear ratio is a ratio of a post-shift rotation speed, which is a rotation speed on the opposite side of the motor M (first spindle unit SP1 side) in the second transmission mechanism T2, to a pre-shift rotation speed, which is a rotation speed on the motor M side in the second transmission mechanism T2, the first transmission gear ratio is smaller than the second transmission gear ratio.

For example, when the first transmission gear ratio of the first transmission mechanism T1 is smaller than 1, the rotation speed on the opposite side of the motor M (first spindle unit SP1 side) become lower than the rotation speed on the motor M side, and a torque increases. When the second transmission gear ratio of the second transmission mechanism T2 is larger than 1, the rotation speed on the opposite side of the motor M (first spindle unit SP1 side) become higher than the rotation speed on the motor M side, and a torque decreases. In this case, the first drive gear 78 has a smaller diameter than the second drive gear 80, and the first drive gear 78 and the motor M can be disposed close to each other. In the present embodiment, the first transmission mechanism T1 is disposed closer to the motor M than the second transmission mechanism T2. More specifically, the motor M is disposed in front of the first drive gear 78 so as to overlap the first drive gear 78 in the upper-lower direction.

The first transmission mechanism T1 and the second transmission mechanism T2 are switched by the connection and disconnection mechanism 50. The connection and disconnection mechanism 50 includes an upper clutch 50U which switches disconnection and connection of power in the first transmission mechanism T1, a lower clutch 50D which switches disconnection and connection of power in the second transmission mechanism T2, and the operator 55 which rotates integrally with the input gear 77.

The upper clutch 50U is a dog clutch including a first engagement element 51 which is an engagement element on the motor M side and a second engagement element 52 which is an engagement element on a first transmission mechanism T1 side. More specifically, the first engagement element 51 is provided above the operator 55 and the input gear 77 so as to rotate integrally with the input gear 77. The second engagement element 52 is provided at a lower part of the first drive gear 78 so as to be engageable with the first engagement element 51 and rotate integrally with the first drive gear 78. The second engagement element 52 and the first drive gear 78 are attached at an upper part of the second spindle 75 of the second spindle unit SP2 so as to be rotatable and immovable relative to the second spindle 75.

The lower clutch 50D is a dog clutch including a third engagement element 53 which is an engagement element on the motor M side and a fourth engagement element 54 which is an engagement element on a second transmission mechanism T2 side. More specifically, the third engagement element 53 is provided below the operator 55 and the input gear 77 so as to rotate integrally with the input gear 77. The fourth engagement element 54 is provided at an upper part of the second drive gear 80 so as to be engageable with the third engagement member 53 and rotate integrally with the second drive gear 80. The fourth engagement element 54 and the second drive gear 80 are attached at a lower part of the second spindle 75 of the second spindle unit SP2 so as to be rotatable and immovable relative to the second spindle 75.

The operator 55 is attached to the input gear 77 so as to rotate integrally with the input gear 77 as described above, and the first engagement member 51 and the third engagement element 53 are attached to an upper portion and a lower portion thereof so as to integrally rotate. The operator 55 is interposed substantially at a center of the second spindle 75, that is, between the first drive gear 78 and the second drive gear 80 in the upper-lower direction. Here, the operator 55 is a screw nut 76 of the second spindle unit SP2. The second spindle unit SP2 includes the second spindle 75 formed with a male thread, and the screw nut 76 (operator 55) formed with a female thread. In accordance with rotation of the input gear 77, the screw nut 76 (operator 55) performs translational motion while rotating along an axis of the second spindle 75.

The screw nut 76 (operator 55) moves upward in the upper-lower direction, which is a moving direction of the translational motion, when the motor M rotates in a first direction (direction of arrow D1 in FIG. 5A), and moves downward in the upper-lower direction when the motor M rotates in a second direction (direction of arrow D2 in FIG. 5B) opposite to the first direction. In this way, a moving direction of the operator 55 can be changed by changing a rotation direction of the motor M.

The connection and disconnection mechanism 50 can take three states of a first gear shift state, a second gear shift state, and a free state by translational motion of the operator 55 along the axis of the second spindle 75.

In the first gear shift state, as illustrated in FIG. 5A, the motor M rotates in the first direction (direction of arrow D1 in FIG. 5A), and the screw nut 76 (operator 55) moves upward. Therefore, the first engagement element 51 and the second engagement element 52 are in a connected state, and the third engagement element 53 and the fourth engagement element 54 are in a disconnected state. In other words, the upper clutch 50U is in an engaged state, and the lower clutch 50D is in a disengaged state. In the first gear shift state, the power of the motor M is transmitted to the output gear 72, the input gear 77, the operator 55, the first engagement element 51, the second engagement element 52, the first drive gear 78, the first driven gear 79, and the first spindle unit SP1.

In the second gear shift state, as illustrated in FIG. 5B, the motor M rotates in the second direction (direction of arrow D2 in FIG. 5B), and the screw nut 76 (operator 55) moves downward. Therefore, the first engagement element 51 and the second engagement element 52 are in a disconnected state, and the third engagement element 53 and the fourth engagement element 54 are in a connected state. In other words, the upper clutch 50U is in a disengaged state, and the lower clutch 50D is in an engaged state. In the second gear shift state, the power of the motor M is transmitted to the output gear 72, the input gear 77, the operator 55, the third engagement element 53, the fourth engagement element 54, the second drive gear 80, the second driven gear 81, and the first spindle unit SP1.

In the free state, as illustrated in FIG. 5C, the first engagement element 51 and the second engagement element 52 are in a disconnected state, and the third engagement element 53 and the fourth engagement element 54 are in a disconnected state. In other words, both the upper clutch 50U and the lower clutch 50D are in a disengaged state. In the free state, the motor M is stopped, and the first driven gear 79 and the second driven gear 81 are rotated by rotation of the first spindle unit SP1, and the rotation of the first spindle unit SP1 is transmitted to the first driven gear 79, the first drive gear 78, and the second engagement element 52 but is not transmitted to the first engagement element 51. Similarly, the rotation of the first spindle unit SP1 is transmitted to the second driven gear 81, the second drive gear 80, and the fourth engagement element 54, but is not transmitted to the third engagement element 53.

In FIGS. 4 to 5C, reference sign D denotes a rotary damper, which applies appropriate resistance to the second spindle 75 of the second spindle unit SP2 so that the screw nut 76 (operator 55) can be reliably translated during rotation of the motor M. The rotary damper D includes a first damper gear 86 provided on a rotary shaft of the rotary damper D, and a second damper gear 87 provided on the second spindle 75 of the second spindle unit SP2 so as to rotate integrally with the second spindle 75 and meshing with the first damper gear 86. The rotary damper D is disposed at a front part of and at an upper part of the bottom plate portion 62 of the transmission case 60 and below the motor M.

In the electric prosthetic leg 1 configured in this way, it is possible to smoothly perform an ascending operation of steps, which has been required to be done one by one by a leg (healthy leg) on a non-prosthetic-leg side, with a passive prosthesis leg including a passive damper in the related art.

Specifically, as illustrated in FIG. 6, large power is required when the knee joint mechanism 30 is stretched from a bent state in a state in which the electric prosthetic leg 1 is applied an applied weight from an outside when the electric prosthetic leg 1 is moved forward to ascend steps.

At this time, as illustrated in FIG. 5A, by rotating the motor M in the first direction (direction of arrow D1 in FIG. 5A), the power of the motor M is transmitted from the output gear 72 to the input gear 77. When the input gear 77 rotates in the second direction (direction of arrow D2 in FIG. 5A), the operator 55 moves upward while rotating around the second spindle 75 of the second spindle unit SP2 and being guided by the second spindle 75. The first engagement element 51 provided above the operator 55 and the input gear 77 is engaged with the second engagement element 52 provided at the lower part of the first drive gear 78, and the connection and disconnection mechanism 50 is brought into the first gear shift state.

When the connection and disconnection mechanism 50 is in the first gear shift state, the power of the motor M is transmitted to the output gear 72, the input gear 77, the operator 55, the first engagement element 51, the second engagement element 52, the first drive gear 78, the first driven gear 79, and the first spindle unit SP1. When the first drive gear 78 rotates together with the input gear 77 in the second direction (direction of arrow D2 in FIG. 5A), the first driven gear 79 rotates in the first direction (the direction of the arrow D1 in FIG. 5A). When the first driven gear 79 rotates in the first direction (direction of arrow D1 in FIG. 5A), the first spindle 73 of the first spindle unit SP1 rotates in the first direction (direction of arrow D1 in FIG. 5A). Accordingly, the sleeve 74 is translated so as to be separated from the transmission T, and the upper-knee member 20 to which the sleeve 74 is attached is rotated about the pivoting portion 35 with respect to the lower-knee member 10 to which the transmission T is attached, and the knee joint mechanism 30 is stretched.

On the other hand, in order to smoothly perform an ascending operation of the steps, as illustrated in FIG. 7, it is necessary to bend (lift up) the knee joint mechanism 30 from a stretched state in a state where the healthy leg is applied an applied weight from the outside, that is, in a state where the electric prosthetic leg 1 is not applied an applied weight from the outside. When the knee joint mechanism 30 is bent from the stretched state, large power is not required, but a quick operation is required.

At this time, as illustrated in FIG. 5B, by rotating the motor M in the second direction (direction of arrow D2 in FIG. 5B), the power of the motor M is transmitted from the output gear 72 to the input gear 77. When the input gear 77 rotates in the first direction (direction of arrow D1 in FIG. 5B), the operator 55 moves downward while rotating around the second spindle 75 of the second spindle unit SP2 and being guided by the second spindle 75. Then, the third engagement element 53 provided below the operator 55 and the input gear 77 is engaged with the fourth engagement element 54 provided at the upper part of the second drive gear 80, and the connection and disconnection mechanism 50 is brought into the second gear shift state.

When the connection and disconnection mechanism 50 is in the second gear shift state, the power of the motor M is transmitted to the output gear 72, the input gear 77, the operator 55, the third engagement element 53, the fourth engagement element 54, the second drive gear 80, the second driven gear 81, and the first spindle unit SP1. When the second drive gear 80 rotates together with the input gear 77 in the first direction (direction of arrow D1 in FIG. 5B), the second driven gear 81 rotates in the second direction (direction of arrow D2 in FIG. 5B). When the second driven gear 81 rotates in the second direction (direction of arrow D2 in FIG. 5B), the first spindle 73 of the first spindle unit SP1 rotates in the second direction (direction of arrow D2 in FIG. 5B). Accordingly, the sleeve 74 is translated so as to approach the transmission T, and the lower-knee member 10 to which the transmission T is attached rotate about the pivoting portion 35 with respect to the upper-knee member 20 to which the sleeve 74 is attached, and the knee joint mechanism 30 is bent.

By bringing the connection and disconnection mechanism 50 into the free state during normal walking, power transmission between the motor M and the transmission T can be interrupted, thereby preventing interference with the walking.

In the electric prosthetic leg 1 configured in this way, the knee joint mechanism 30 can be stretched and bent via the transmission T which transmits the power of the motor M. The motor M is controlled by a control system 100 to be described later. A pivoting range of the knee joint mechanism 30 is limited to 180° or less. In a state where the knee joint mechanism 30 is stretched, the angle formed by the lower-knee member 10 and the upper-knee member 20 is about 180°, and in a state where the knee joint mechanism 30 is bent, the formed angle is less than 180°.

Since the transmission T includes two power transmission paths having different transmission gear ratios, the operation speed and generated power of stretching and bending of the knee joint mechanism 30 can be switched. In particular, a required operation speed and generated power are different between a time of bending and a time of stretching the knee joint mechanism 30 when ascending steps, and the power transmission path can be changed between the time of bending and the time of stretching the knee joint mechanism 30.

Since the connection and disconnection mechanism 50 interposed between the motor M and the transmission T includes the upper clutch 50U interposed between the motor M and the first transmission mechanism T1 and the lower clutch 50D interposed between the motor M and the second transmission mechanism T2, the two power transmission paths can be appropriately switched.

In particular, since the expansion and contraction device 40 includes the second spindle unit SP2 which converts the rotational power output from the motor M into the translational motion of the operator 55, the operator 55 can be controlled by one motor M and the stretching and bending of the knee joint mechanism 30 can be controlled. Further, since the transmission T is configured to be switched to the first gear shift state, the second gear shift state, and the free state by one operator 55, it is possible to avoid the two power transmission paths from being connected at the same time.

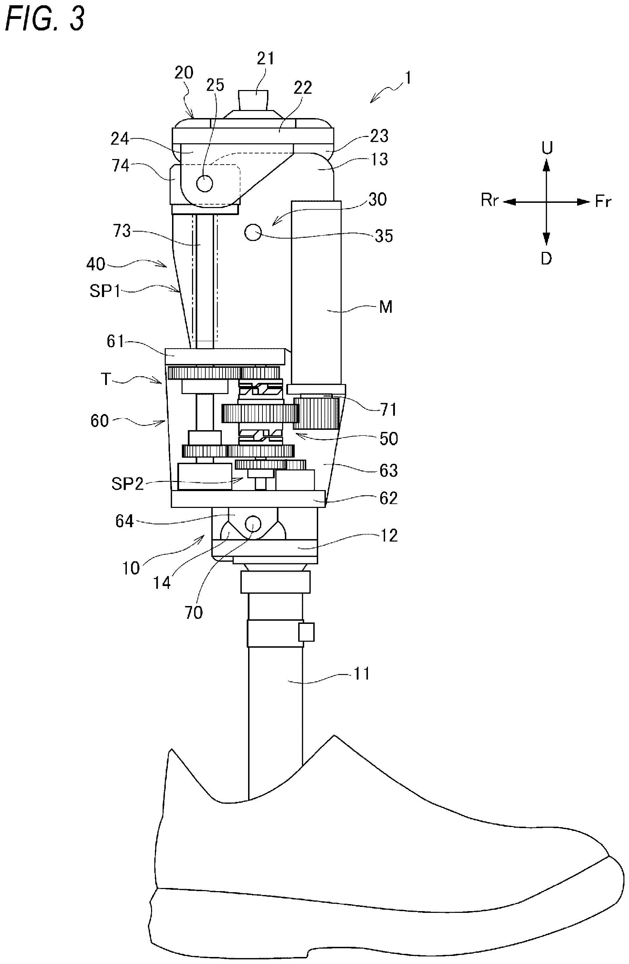

In a state where the knee joint mechanism 30 is stretched, when a region in which the angle formed by the lower-knee member 10 and the upper-knee member 20 is less than 180° (region on rear (calf) side of line connecting pivoting portion 35 and lower swing portion 70 in FIG. 3) is referred to as a narrow angle region (region on rear (calf) side of line connecting pivoting portion 35 and lower swing portion 70 in FIG. 3) and a region in which the angle formed by the lower-knee member 10 and the upper-knee member 20 is 180° or more (region on front (shin) side of line connecting pivoting portion 35 and lower swing portion 70 in FIG. 3) is referred to as a wide angle region (region on front (shin) side of line connecting pivoting portion 35 and lower swing portion 70 in FIG. 3), the first spindle unit SP1 is disposed in the narrow angle region, when the electric prosthetic leg 1 is viewed along a pivoting axis of the pivoting portion 35 of the knee joint mechanism 30. On the other hand, the motor M is disposed in the wide angle region. By disposing the first spindle unit SP1 in the narrow angle region and disposing the motor M in the wide angle region in this manner, the first spindle unit SP1 and the motor M can be disposed in a balanced manner with the pivoting portion 35 of the knee joint mechanism 30 interposed therebetween. Further, the transmission T and the connection and disconnection mechanism 50 are disposed between the motor M and the first spindle unit SP1. Therefore, the motor M, the transmission T, the connection and disconnection mechanism 50, and the first spindle unit SP1 can be collectively arranged.

<Second Embodiment>

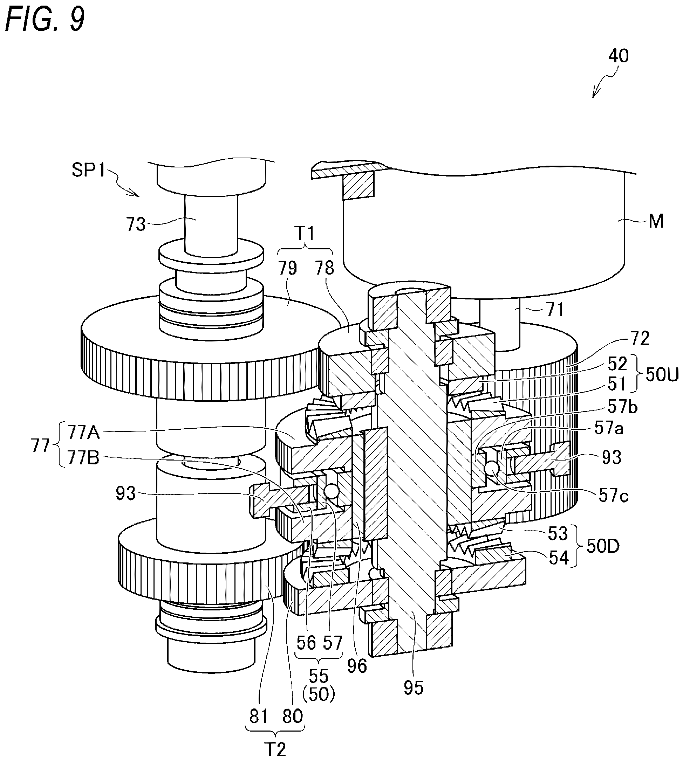

The electric prosthetic leg 1 according to a second embodiment is different from the electric prosthetic leg 1 according to the first embodiment in a configuration of the expansion and contraction device 40. More specifically, in the expansion and contraction device 40 according to the first embodiment, the translational motion of the operator 55 is implemented by converting the rotational power output from the motor M by the second spindle unit SP2. On the other hand, in the expansion and contraction device 40 according to the second embodiment, the translational motion of the operator 55 is implemented by a clutch actuator ACT which is a drive source different from the motor M and a clutch fork 90 which transmits power of the clutch actuator ACT to the operator 55. In the following description, the expansion and contraction device 40 of the electric prosthetic leg 1 according to the second embodiment will be described with reference to FIGS. 8 and 9. The same or equivalent configurations as those of the expansion and contraction device 40 of the electric prosthetic leg 1 according to the first embodiment will be denoted by the same reference signs in the drawings, the description thereof will be omitted, and only differences will be described.

As illustrated in FIGS. 8 and 9, the expansion and contraction device 40 according to the second embodiment includes the motor M which outputs rotational power, the transmission T which transmits power of the motor M, the first spindle unit SP1 which is connected to the transmission T in a power transmittable manner and converts the rotational power output from the transmission T into translational motion, the connection and disconnection mechanism 50 which is interposed between the motor M and each of the first transmission mechanism T1 and the second transmission mechanism T2 of the transmission T, a support shaft 95 which is disposed parallel to the output shaft 71 of the motor M and the first spindle 73 of the first spindle unit SP1 and supports the connection and disconnection mechanism 50, the clutch actuator ACT which performs translational motion, and the clutch fork 90 which transmits power of the clutch actuator ACT to the operator 55 of the connection and disconnection mechanism 50.

The clutch actuator ACT is a power source different from the motor M, and performs translational motion along an axial direction (upper-lower direction) of the support shaft 95 as indicated by an arrow Y1 in FIG. 8.

The clutch fork 90 is configured to be swingable around a swing shaft 65 as indicated by an arrow Y2 in FIG. 8, with one end portion of the clutch fork 90 connected to the clutch actuator ACT, and an intermediate portion of the clutch fork 90 supported by the swing shaft 65. The other end portion of the clutch fork 90 is provided with an arm 92 branched in two from a branch 91 located on an opposite side of the clutch actuator ACT with respect to the swing shaft 65 and extending in opposite directions to each other in an arc shape. A coupling pin 93 to be engaged with a slide clutch 56 to be described later is provided at a tip end of each arm 92. Accordingly, when the clutch actuator ACT performs translational motion, the clutch fork 90 swings around the swing shaft 65, and the coupling pin 93 of the clutch fork 90 swings up and down.

The transmission T includes the output gear 72 provided on the output shaft 71 of the motor M, a first input gear 77A and a second input gear 77B provided substantially in a central portion of the support shaft 95 and meshing with the output gear 72, the first transmission mechanism T1, and the second transmission mechanism T2. The first input gear 77A and the second input gear 77B constitute the input gear 77.

The first transmission mechanism T1 includes the first drive gear 78 immovably provided on an upper side of the support shaft 95, and the first driven gear 79 provided on the first spindle 73 of the first spindle unit SP1 so as to rotate integrally with the first spindle 73 and meshing with the first drive gear 78.

The second transmission mechanism T2 includes the second drive gear 80 immovably provided on a lower side of the support shaft 95, and the second driven gear 81 provided on the first spindle 73 of the first spindle unit SP1 so as to rotate integrally with the first spindle 73 and meshing with the second drive gear 80.

The transmission gear ratios of the first transmission mechanism T1 and the second transmission mechanism T2 are the same as those in the first embodiment, and therefore the description thereof is omitted here. The first transmission mechanism T1 and the second transmission mechanism T2 are switched by the connection and disconnection mechanism 50. The connection and disconnection mechanism 50 includes the upper clutch 50U interposed between the motor M and the first transmission mechanism T1, the lower clutch 50D interposed between the motor M and the second transmission mechanism T2, and the operator 55.

The upper clutch 50U is a dog clutch including a first engagement element 51 which is an engagement element on the motor M side and a second engagement element 52 which is an engagement element on a first transmission mechanism T1 side. More specifically, the first engagement element 51 is provided at an upper part of the first input gear 77A so as to rotate integrally with the first input gear 77A. The second engagement element 52 is provided at a lower part of the first drive gear 78 so as to be engageable with the first engagement element 51 and rotate integrally with the first drive gear 78. The second engagement element 52 and the first drive gear 78 are attached at an upper part of the support shaft 95 so as to be rotatable and immovable relative to the support shaft 95.

The lower clutch 50D is a dog clutch including a third engagement element 53 which is an engagement element on the motor M side and a fourth engagement element 54 which is an engagement element on a second transmission mechanism T2 side. More specifically, the third engagement element 53 is provided at a lower part of the second input gear 77B so as to rotate integrally with the second input gear 77B. The fourth engagement element 54 is provided at an upper part of the second drive gear 80 so as to be engageable with the third engagement member 53 and rotate integrally with the second drive gear 80. The fourth engagement element 54 and the second drive gear 80 are attached to a lower part of the support shaft 95 so as to be rotatable and immovable relative to the support shaft 95.

The operator 55 includes annular slide clutches 56 which are constantly engaged with the coupling pins 93 of the clutch fork 90, and a bearing 57 which performs translational motion together with the slide clutches 56, and is disposed between the first input gear 77A and the second input gear 77B in the upper-lower direction.

The slide clutch 56 is provided with a coupling hole with which the coupling pin 93 of the clutch fork 90 is engaged. The coupling hole absorbs swing of the clutch fork 90 and converts the swing into translational motion of the slide clutch 56 in the upper-lower direction.

The bearing 57 includes an outer ring 57a non-rotatably supported by the slide clutch 56, an inner ring 57b configured to rotate integrally with the first input gear 77A provided with the first engagement element 51 and the second input gear 77B provided with the third engagement element 53, and a rolling body 57c disposed between the outer ring 57a and the inner ring 57b and allowing relative rotation between the outer ring 57a and the inner ring 57b.

The inner ring 57b is supported by an outer peripheral portion of a support flange 96 which is key-coupled to the support shaft 95 together with the first input gear 77A and the second input gear 77B so as to be able to be translated in the upper-lower direction.

The slide clutch 56 and the bearing 57 (operator 55) move upward along the support shaft 95 when one end of the clutch fork 90 moves downward by the clutch actuator ACT, and move downward along the support shaft 95 when the one end of the clutch fork 90 moves upward by the clutch actuator ACT. A predetermined gap is provided between the first input gear 77A and each of the slide clutch 56 and the outer ring 57a and in the upper-lower direction, so that the first input gear 77A does not interfere with the slide clutch 56 and the outer ring 57a which are non-rotating members. Similarly, a predetermined gap is provided between the second input gear 77B and each of the slide clutch 56 and the outer ring 57a in the upper-lower direction, so that the second input gear 77B does not interfere with the slide clutch 56 and the outer ring 57a which are non-rotating members.

The connection and disconnection mechanism 50 can take three states of the first gear shift state, the second gear shift state, and the free state by the slide clutch 56 and the bearing 57 (operator 55) performing translational motion in the upper-lower direction along an axis of the support shaft 95.

In the first gear shift state, the slide clutch 56 and the bearing 57 (operator 55) move upward, so that the first engagement element 51 and the second engagement element 52 are in a connected state and the third engagement element 53 and the fourth engagement element 54 are in a disconnected state as in FIG. 5A. In other words, the upper clutch 50U is engaged and the lower clutch 50D is disengaged. In the first gear shift state, the power of the motor M is transmitted to the output gear 72, the first input gear 77A (inner ring 57b and second input gear 77B), the first engagement element 51, the second engagement element 52, the first drive gear 78, the first driven gear 79, and the first spindle unit SP1.

In the second gear shift state, the slide clutch 56 and the bearing 57 (operator 55) move downward, so that the first engagement element 51 and the second engagement element 52 are in a disconnected state and the third engagement element 53 and the fourth engagement element 54 are in a connected state as in FIG. 5B. In other words, the upper clutch 50U is disengaged and the lower clutch 50D is engaged. In the second gear shift state, the power of the motor M is transmitted to the output gear 72, the second input gear 77B (inner ring 57b and first input gear 77A), the third engagement element 53, the fourth engagement element 54, the second drive gear 80, the second driven gear 81, and the first spindle unit SP1.

In the free state, similarly to FIG. 5C, the first engagement element 51 and the second engagement element 52 are in a disconnected state, and the third engagement element 53 and the fourth engagement element 54 are in a disconnected state. In other words, the upper clutch 50U is disengaged and the lower clutch 50D is disengaged. In the free state, the motor M is stopped, and the first driven gear 79 and the second driven gear 81 are rotated by rotation of the first spindle unit SP1, and the rotation of the first spindle unit SP1 is transmitted to the first driven gear 79, the first drive gear 78, and the second engagement element 52 but is not transmitted to the first engagement element 51. Similarly, the rotation of the first spindle unit SP1 is transmitted to the second driven gear 81, the second drive gear 80, and the fourth engagement element 54, but is not transmitted to the third engagement element 53.

In the electric prosthetic leg 1 configured as described above, as in the first embodiment, the knee joint mechanism 30 can be stretched and bent via the transmission T which transmits the power of the motor M, and an operation and effect described in the electric prosthetic leg 1 according to the first embodiment can be obtained. In the electric prosthetic leg 1 according to the second embodiment, the operator 55 can be switched more stably by switching the operator 55 using the clutch actuator ACT which is a power source different from the motor M for stretching and bending the knee joint mechanism 30.

<Step Ascending Control>

Next, step ascending control using the electric prosthetic leg 1 according to the first embodiment and the second embodiment described above will be described by taking the electric prosthetic leg 1 according to the second embodiment as an example. In the following description, a leg to which no applied weight is applied from an outside, in other words, a leg which does not support a body weight is referred to as an idling leg, and a leg to which an applied weight is applied from the outside, in other words, a leg which supports the body weight is referred to as a standing leg. Although a weight (own weight) due to gravity of the electric prosthetic leg 1 normally acts on the electric prosthetic leg 1, it is noted that the own weight is not included in the applied weight from an outside.

[First Example]

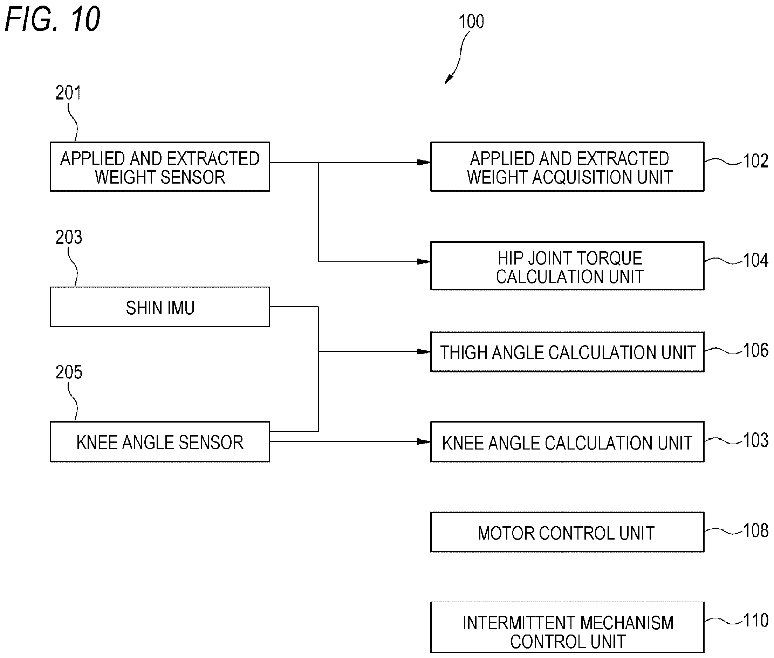

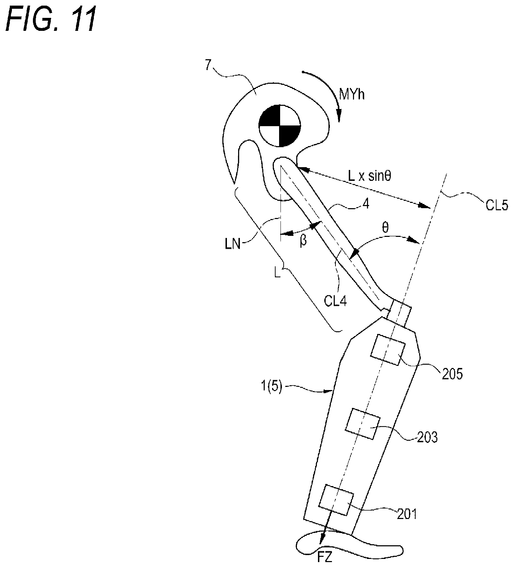

As illustrated in FIGS. 10 and 11, the electric prosthetic leg 1 is provided with an applied and extracted weight sensor 201 which is a load sensor provided at a lower portion (for example, leg portion 11) of the lower-knee member 10, a shin inertial measurement unit (IMU) 203 which is a posture sensor provided at the lower-knee member 10 and capable of detecting an angle of the lower-knee member 10 with respect to the ground (ground angle), and a knee angle sensor 205 which is a rotation angle sensor for detecting an angle formed by the lower-knee member 10 and the upper-knee member 20.

The control system 100 of the electric prosthetic leg 1 includes an applied and extracted weight acquisition unit 102 which acquires an applied weight FZ applied to the lower-knee member 10, a knee angle calculation unit 103 which acquires a knee angle θ which is an angle between a center line CL5 of the lower leg 5 and a center line CL4 of the thigh 4, a hip joint torque calculation unit 104 which calculates a hip joint torque MYh which acts on a hip joint 7 due to the applied weight FZ applied to the lower-knee member 10, a thigh angle calculation unit 106 which calculates a thigh angle β which is an angle of the thigh 4 with respect to a virtual line LN extending in a vertical direction through the hip joint 7, a motor control unit 108 which controls the motor M, and an connection and disconnection mechanism control unit 110 which controls a state of the connection and disconnection mechanism 50. In the first embodiment, it is noted that the motor control unit 108 also serves as the connection and disconnection mechanism control unit 110.

The applied and extracted weight acquisition unit 102 acquires the applied weight FZ, which is an axial force applied to the lower-knee member 10, from the applied and extracted weight sensor 201. The applied weight FZ applied to the lower-knee member 10 may be a detection value of the applied and extracted weight sensor 201 or a predicted value. In the present embodiment, a positive direction is a direction in which the applied weight FZ applied to the lower-knee member 10 is in a tensile direction.

The knee angle calculation unit 103 obtains the knee angle θ by subtracting an angle detected by the knee angle sensor 205 from 180°. The knee angle θ is 0° when the center line CL5 of the lower leg 5 and the center line CL4 of the thigh 4 coincide with each other, and becomes larger in the positive direction as the knee joint mechanism 30 bends.

The hip joint torque calculation unit 104 multiplies the applied weight FZ applied to the lower-knee member 10 by a distance from the center line CL5 of the lower leg 5 to the hip joint 7 (length of thigh 4) to obtain the hip joint torque MYh. When the length of the thigh 4 of a user is L and the knee angle is θ, the hip joint torque MYh is expressed by the following Formula (1). The positive direction is a direction in which the hip joint 7 is pivoted forward (clockwise in FIG. 11) by the hip joint torque MYh. The length L of the thigh 4 is preferably registered in advance in a memory (not illustrated) and rewritable according to the user.

$$MYh = FZ \times L \times \sin\theta \qquad (1)$$

The thigh angle calculation unit 106 calculates the thigh angle β based on output values of the shin IMU 203 and the knee angle sensor 205. The thigh angle β is a positive value when the thigh 4 is behind the virtual line LN extending in the vertical direction through the hip joint 7, and is a negative value when the thigh 4 is ahead of the virtual line LN.

The motor control unit 108 controls drive and stop of the motor M and an output when the motor M is driven. The step ascending control will be described later.

The connection and disconnection mechanism control unit 110 switches the connection and disconnection mechanism 50 to the first gear shift state, the second gear shift state, and the free state described above by switching the operator 55 based on the applied weight FZ acquired by the applied and extracted weight acquisition unit 102.

Specifically, when the applied and extracted weight acquisition unit 102 acquires or predicts a transition from a state in which no applied weight is applied to a state in which an applied weight is applied, in other words, when the electric prosthetic leg 1 transitions from an idling leg to a standing leg or the transition is predicted, the connection and disconnection mechanism control unit 110 switches the upper clutch 50U out of the upper clutch 50U and the lower clutch 50D of the connection and disconnection mechanism 50 to an engaged state and sets the state to the first gear shift state. Accordingly, in a case of the standing leg where the knee joint mechanism 30 is stretched from the bent state, power can be transmitted via the first transmission mechanism T1 having a small transmission gear ratio, and large power can be transmitted to the electric prosthetic leg 1 in a state of being applied an applied weight from the outside.

On the other hand, when the applied and extracted weight acquisition unit 102 acquires or predicts a transition from a state in which an applied weight is applied to a state in which no applied weight is applied, in other words, when the electric prosthetic leg 1 transitions from the standing leg to the idling leg or the transition is predicted, the connection and disconnection mechanism control unit 110 switches the lower clutch 50D out of the upper clutch 50U and the lower clutch 50D of the connection and disconnection mechanism 50 to an engaged state and sets the state to the second gear shift state. Accordingly, in a case of the idling leg where the knee joint mechanism 30 is bent from the stretched state, power can be transmitted via the second transmission mechanism T2 having a large transmission gear ratio, and the knee joint mechanism 30 can be quickly bent in a state where an applied weight is not applied to the electric prosthetic leg 1 from the outside.

The control system 100 may include a traveling direction acquisition unit which acquires a traveling direction of the user of the electric prosthetic leg 1. When the traveling direction acquisition unit detects an angular velocity toward an upper side in the vertical direction, such as when ascending steps or ascending a slope, based on a detection value of the shin IMU 203, the step ascending control may be executed, or the step ascending control may be executed based on an input of the user.

Hereinafter, a control flow of a first example of the step ascending control will be described.

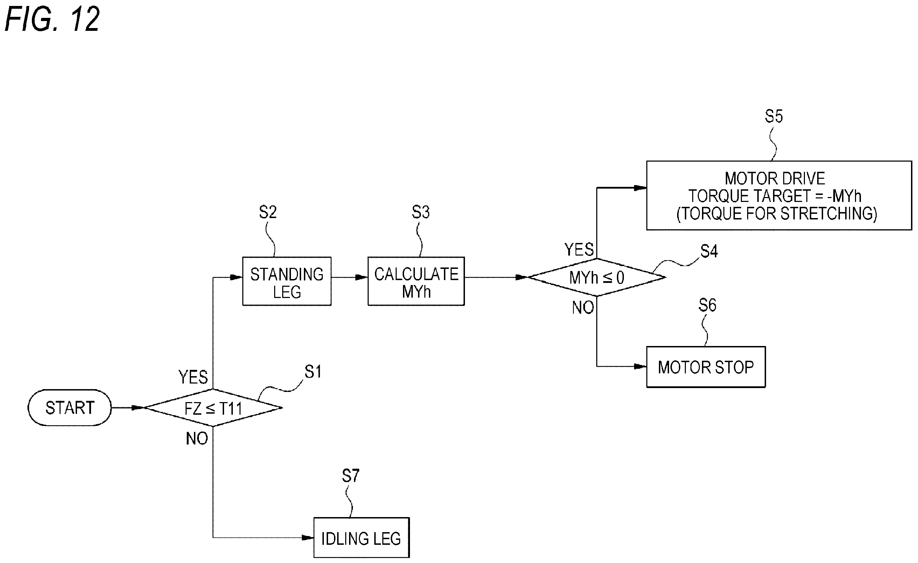

As illustrated in FIG. 12, in the step ascending control, the control system 100 performs a standing leg or idling leg determination processing of determining whether the electric prosthetic leg 1 is a standing leg or an idling leg (S1). Specifically, the control system 100 performs standing leg or idling leg determination based on the applied weight FZ acquired by the applied and extracted weight acquisition unit 102. As described above, when the applied weight FZ applied to the lower-knee member 10 is in the tensile direction, the positive direction is taken. Therefore, when the electric prosthetic leg 1 is the idling leg, the applied weight FZ takes a positive value due to the own weight of the electric prosthetic leg 1. Then, when the electric prosthetic leg 1 lands and an applied weight is applied to the electric prosthetic leg 1 in a compression direction, the applied weight FZ approaches zero and takes a negative value when the applied weight further increases.

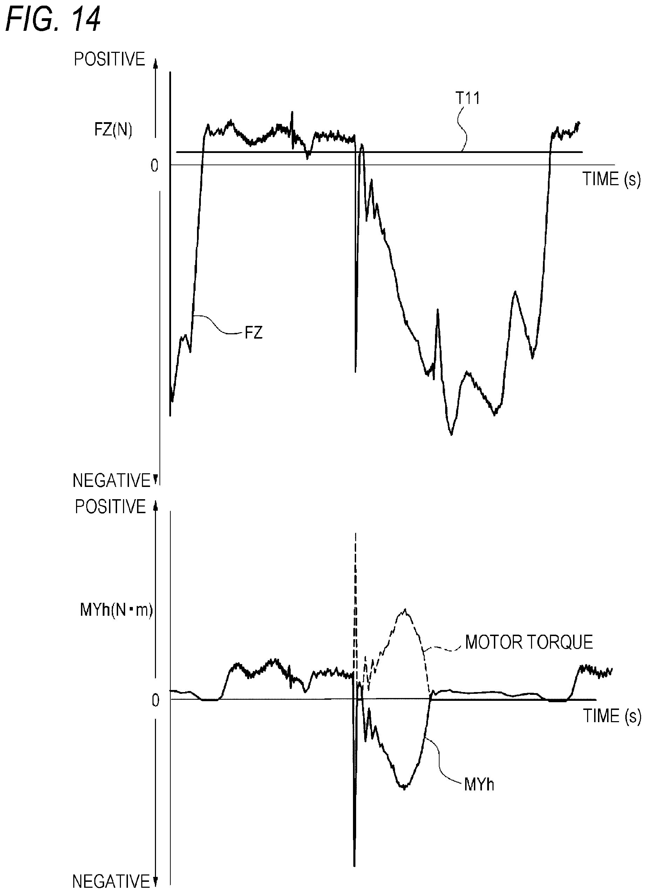

FIG. 14 is a graph illustrating the applied weight FZ and the hip joint torque MYh at the time of ascending steps, in which a vertical axis of an upper graph of FIG. 14 indicates the applied weight FZ (N), a vertical axis of a lower graph of FIG. 14 indicates the hip joint torque MYh (N·m), and horizontal axes of the upper graph and the lower graph of FIG. 14 indicate time (s).

Referring to FIGS. 12 and 14, the control system 100 determines whether the applied weight FZ is equal to or less than a threshold value T11 (N), the threshold value T11 being a value between 0 (N) and a value when only the own weight acts on the electric prosthetic leg 1 (S1). When the applied weight FZ is equal to or less than the threshold value T11 (YES in S1), it is determined that the electric prosthetic leg 1 is the standing leg (S2). On the other hand, when the applied weight FZ is larger than the threshold value T11 (NO in step S1), it is determined that the electric prosthetic leg 1 is the idling leg (S7). The determination of the standing leg and the idling leg may be determined based on an input of the user. Any units such as a switch, a button, or a voice can be adopted for the input by the user.

When the electric prosthetic leg 1 is the standing leg, the hip joint torque calculation unit 104 calculates the hip joint torque MYh acting on the hip joint 7 by the applied weight FZ (S3). When the electric prosthetic leg 1 is applied an applied weight in the compression direction, a hip joint torque which pivots the hip joint 7 backward (counterclockwise in FIG. 11) is generated. The hip joint torque calculation unit 104 acquires the hip joint torque MYh according to Formula (1) based on the knee angle $\theta$ based on the angle formed by the lower-knee member 10 and the upper-knee member 20, the length L of the thigh 4, and the applied weight FZ acquired by the applied and extracted weight acquisition unit 102.

When the calculated hip joint torque MYh is equal to or less than 0 (N·m) (YES in S4), that is, when the torque acts in the direction in which the hip joint 7 is pivoted backward (counterclockwise in FIG. 11) by the applied weight (early stage of standing leg), as indicated by a dotted line in the lower graph of FIG. 14, the motor control unit 108 performs current control of the motor M with a torque in an opposite direction having the same magnitude as the hip joint torque MYh as a control target. Accordingly, a motor torque and the hip joint torque MYh are balanced, and the knee joint mechanism 30 can be stretched (S5). By controlling the motor torque generated by the motor M based on the hip joint torque calculated by the hip joint torque calculation unit 104 in this manner, appropriate motor torque for stretching can be set.

On the other hand, when the calculated hip joint torque MYh is larger than 0 (NO in S4), that is, when the torque acts in a direction in which the hip joint 7 is pivoted forward (counterclockwise in FIG. 11) by the applied weight (from middle stage to latter stage of standing leg), the motor M is stopped as illustrated in FIG. 14 (S6). By maintaining engagement of the upper clutch 50U in the first gear shift state even when the motor M is stopped, sliding resistance acts on the knee joint mechanism 30.

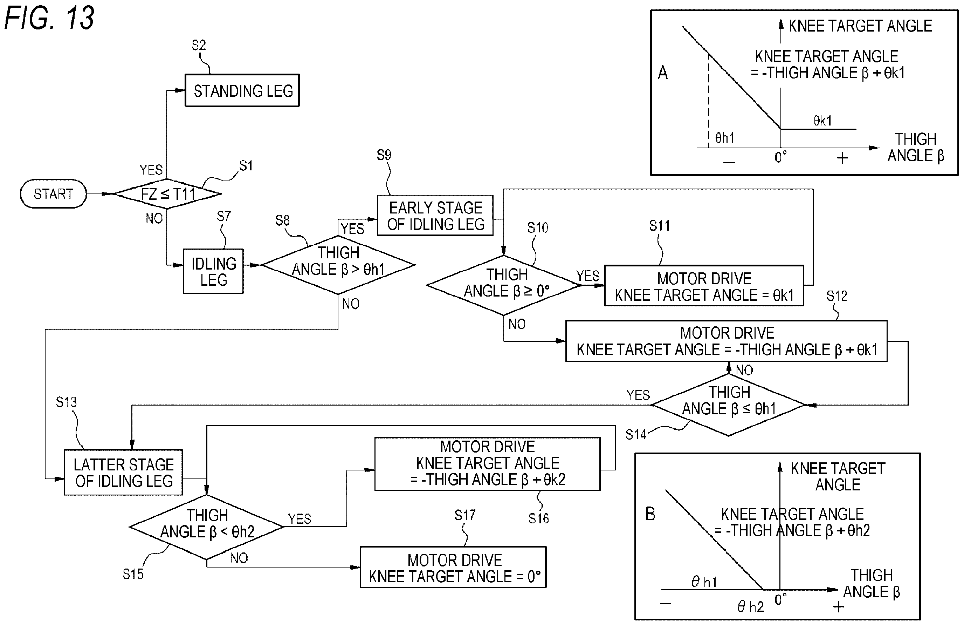

When the electric prosthetic leg 1 is the idling leg, as illustrated in FIG. 13, the control system 100 determines whether the thigh angle $\beta$ is larger than a threshold value $\theta$h1 ($\theta$h1 is negative), the thigh angle $\beta$ dividing the early stage of the idling leg and the latter stage of the idling leg (S8). When the thigh angle $\beta$ is larger than the threshold value $\theta$h1 (YES in S8), it is determined to be the early stage of the idling leg, that is, a status in which the user lifts up the thigh 4 and bends the knee joint mechanism 30 (S9). In the early stage of the idling leg, subsequently, it is determined whether the thigh angle $\beta$ is equal to or larger than 0° (S10). When the thigh angle $\beta$ is equal to or larger than 0° (YES in S10), a target of the knee angle $\theta$ (hereinafter, a knee target angle) is set to $\theta$k1 (a positive value), and the motor M is current-controlled until the thigh angle $\beta$ becomes smaller than 0° (S11).

When the thigh angle $\beta$ is smaller than 0° in step S10 (NO in S10), the knee target angle is set as a value obtained by multiplying the thigh angle $\beta$ by -1 and adding $\theta$k1, the motor M is current-controlled (S12). A in FIG. 13 is a graph illustrating the knee target angle in steps S11 and S12.

On the other hand, in step S8, when the thigh angle $\beta$ is equal to or less than the threshold value $\theta$h1 (NO in S8), and when the knee joint mechanism 30 is bent from step S12 and the thigh angle $\beta$ becomes equal to or less than the threshold value $\theta$h1 (YES in S14), it is determined to be in the latter stage of the idling leg, that is, the knee joint mechanism 30 is to be stretched when the user lowers the thigh 4 (S13). In the latter stage of the idling leg, subsequently, it is determined whether the thigh angle β is smaller than a threshold value θh2 (θh2 is negative and θh2 > θh1) (S15). When the thigh angle β is smaller than θh2 (YES in S15), the knee target angle is set to a value obtained by multiplying the thigh angle β by -1 and adding θh2, and the motor M is current-controlled until the thigh angle β becomes equal to or larger than θh2 (S16). In step S15, when the thigh angle β is equal to or larger than θh2 (NO in S15), the knee target angle is set to 0° and the motor M is current-controlled (S17). B in FIG. 13 is a graph illustrating the knee target angle in steps S16 and S17. The motor control unit 108 preferably performs feedback control based on the knee angle θ and the knee target angle acquired by the knee angle calculation unit 103 in steps S11, S12, S16, and S17.

[Second Example]

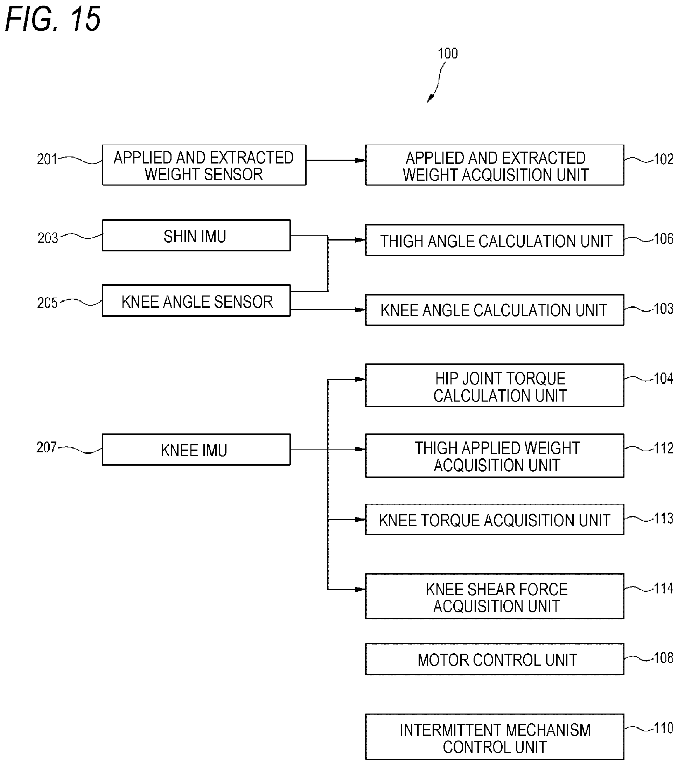

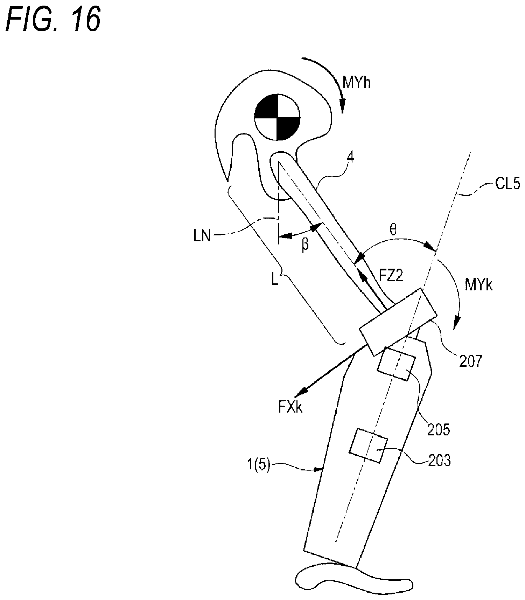

As illustrated in FIGS. 15 and 16, the electric prosthetic leg 1 used in the step ascending control according to the second example includes a force sensor 207 in place of the applied and extracted weight sensor 201 described above. That is, the electric prosthetic leg 1 according to the second example includes the shin IMU 203, the knee angle sensor 205, and the force sensor 207. As illustrated in FIG. 16, the force sensor 207 is configured to detect at least an applied weight FZ2 which is an axial force applied to the thigh 4, a knee shear force FXk acting in a direction orthogonal to the applied weight FZ2, and a knee torque MYk which is a pivoting force of the knee joint mechanism 30 around the pivoting portion 35.

The control system 100 of the electric prosthetic leg 1 includes the applied and extracted weight acquisition unit 102 which acquires the applied weight FZ applied to the lower-knee member 10, the knee angle calculation unit 103 which acquires the knee angle θ which is the angle between the center line CL5 of the lower leg 5 and the center line CL4 of the thigh 4, the thigh angle calculation unit 106 which calculates the thigh angle β which is the angle of the thigh 4 with respect to the virtual line LN extending in the vertical direction through the hip joint 7, a thigh applied weight acquisition unit 112 which acquires the applied weight FZ2 applied to the thigh 4, a knee torque acquisition unit 113 which acquires the knee torque MYk, a knee shear force acquisition unit 114 which acquires the knee shear force FXk applied to the thigh 4, the hip joint torque calculation unit 104 which calculates the hip joint torque MYh which acts on the hip joint 7 by the knee torque MYk and the knee shear force FXk, the motor control unit 108 which controls the motor M, and the connection and disconnection mechanism control unit 110 which controls a state of the connection and disconnection mechanism 50.

The applied and extracted weight acquisition unit 102, the knee angle calculation unit 103, the thigh angle calculation unit 106, and the motor control unit 108 are similar as those in the first example, and description thereof will be omitted.

The thigh applied weight acquisition unit 112, the knee torque acquisition unit 113, and the knee shear force acquisition unit 114 respectively acquire the applied weight FZ2, the knee torque MYk, and the knee shear force FXk from the force sensor 207. In the present embodiment, the positive direction is a direction when the applied weight FZ2 applied to the thigh 6 is in the compression direction.

The hip joint torque calculation unit 104 adds a value obtained by multiplying the length L of the thigh 4 by the knee shear force FXk to the knee torque MYk to obtain the hip joint torque MYh. In this example, the hip joint torque MYh is expressed by the following Formula (2). The positive direction is a direction in which the hip joint 7 is pivoted forward (clockwise in FIG. 16) by the hip joint torque MYh.

$$MYh = MYk + L \times FXk \quad (2)$$

The connection and disconnection mechanism control unit 110 switches the connection and disconnection mechanism 50 to the first gear shift state, the second gear shift state, and the free state described above based on the applied weight FZ2 acquired by the thigh applied weight acquisition unit 112.

Specifically, when the thigh applied weight acquisition unit 112 acquires or predicts a transition from a state in which no applied weight is applied from the outside to a state in which an applied weight is applied, in other words, when the electric prosthetic leg 1 transitions from an idling leg to a standing leg or the transition is predicted, the upper clutch 50U out of the upper clutch 50U and the lower clutch 50D of the connection and disconnection mechanism 50 is switched to an engaged state and sets the state to the first gear shift state. Accordingly, in a case of the standing leg where the knee joint mechanism 30 is stretched from the bent state, power can be transmitted via the first transmission mechanism T1 having a small transmission gear ratio, and large power can be transmitted to the electric prosthetic leg 1 in a state of being applied an applied weight from the outside.

On the other hand, when the thigh applied weight acquisition unit 112 acquires or predicts a transition from a state in which an applied weight is applied from the outside to a state in which no applied weight is applied, in other words, when the electric prosthetic leg 1 transitions from the standing leg to the idling leg or the transition is predicted, the lower clutch 50D out of the upper clutch 50U and the lower clutch 50D of the connection and disconnection mechanism 50 is switched to an engaged state and sets the state to the second gear shift state. Accordingly, in a case of the idling leg where the knee joint mechanism 30 is bent from the stretched state, power can be transmitted via the second transmission mechanism T2 having a large transmission gear ratio, and the knee joint mechanism 30 can be quickly bent in a state where an applied weight is not applied to the electric prosthetic leg 1 from the outside.

The control system 100 may include a traveling direction acquisition unit which acquires a traveling direction of the user of the electric prosthetic leg 1 as in the first example.

Hereinafter, a control flow of the step ascending control according to the second example will be described.

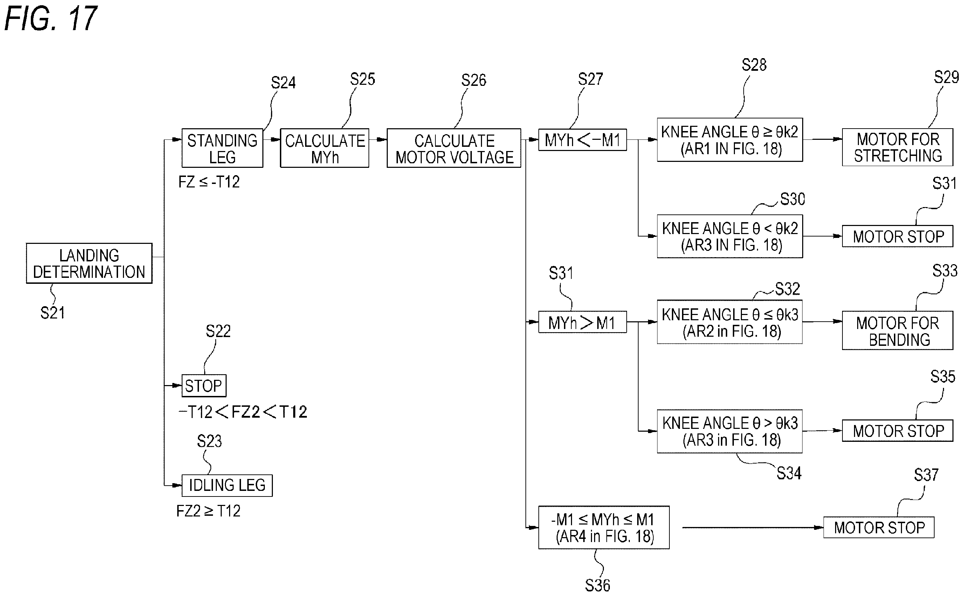

As illustrated in FIG. 17, the control system 100 performs landing determination for determining whether the electric prosthetic leg 1 has landed in the step ascending control (S21). Specifically, the control system 100 performs landing determination based on the applied weight FZ2 acquired by the thigh applied weight acquisition unit 112. When the electric prosthetic leg 1 is an idling leg, the applied weight FZ2 takes a positive value due to its own weight (component force of applied weight FZ). Then, when the electric prosthetic leg 1 lands and an applied weight is applied to the electric prosthetic leg 1 in the compression direction, the applied weight FZ2 approaches zero and takes a negative value when the applied weight further increases.

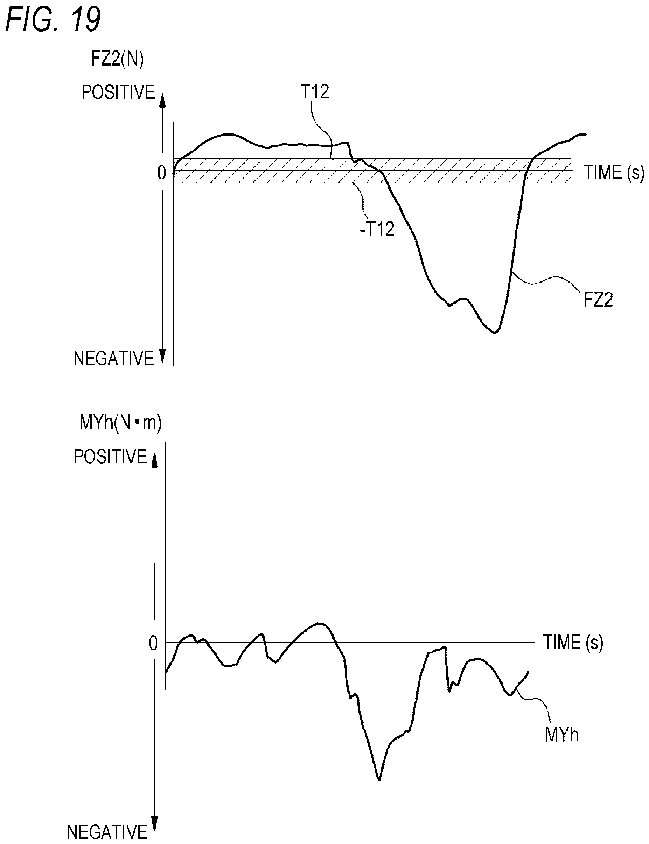

FIG. 19 is a graph illustrating the applied weight FZ2 and the hip joint torque MYh at the time of ascending steps, in which a vertical axis of an upper graph of FIG. 19 indicates the applied weight FZ2 (N), a vertical axis of a lower graph of FIG. 19 indicates the hip joint torque MYh (N·m), and horizontal axes of the upper graph and the lower graph of FIG. 19 indicate time (s).

Referring to FIGS. 17 and 19, when -T12 < FZ2 < T12 is satisfied in a region where a behavior of the applied weight FZ2 is unstable across 0 (N), the control system 100 stops the motor M without determining whether the electric prosthetic leg 1 is a standing leg or an idling leg (S22). When the applied weight FZ2 is equal to or larger than the upper threshold value T12, it is determined that the electric prosthetic leg 1 is the idling leg (S23). Since a control flow when the electric prosthetic leg 1 is the idling leg is similar as that in the first example, the description thereof is omitted here.

When the applied weight FZ2 is equal to or less than the lower threshold value -T12, it is determined that the electric prosthetic leg 1 lands and the electric prosthetic leg 1 is the standing leg (S24). The landing determination may be made based on an input of the user.

When the electric prosthetic leg 1 is the standing leg, the hip joint torque calculation unit 104 calculates the hip joint torque MYh acting on the hip joint 7 based on the knee torque MYk and the knee shear force FXk (S25). The hip joint torque calculation unit 104 acquires the hip joint torque MYh based on a sum of the knee shear force FXk, the length L of the thigh 4, and the knee torque MYk according to Formula (2).

Subsequently, the motor control unit 108 calculates a motor voltage (S26). The motor voltage is acquired according to a formula based on the hip joint torque MYh, a map indicating a relationship between the hip joint torque MYh and the motor voltage, or the like. That is, by setting the motor voltage and controlling the motor torque generated by the motor M based on the hip joint torque calculated by the hip joint torque calculation unit 104, the motor control unit 108 can appropriately set the motor torque for stretching.

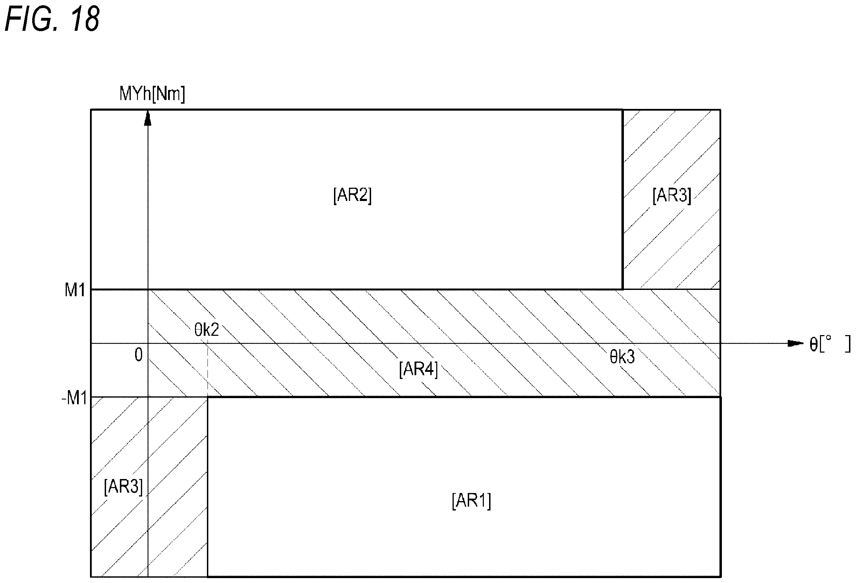

When the hip joint torque MYh is smaller than a threshold value -M1 (S27), that is, when a torque acts in a direction of pivoting the hip joint 7 backward (counterclockwise in FIG. 16), if the knee angle θ is equal to or larger than the threshold value θk2 (S28, region AR1 in FIG. 18), the motor voltage calculated in step S26 is applied (S29). Accordingly, a motor torque and the hip joint torque MYh are balanced, and the knee joint mechanism 30 can be stretched. The threshold value θk2 is a critical value for avoiding contact between mechanical elements inside the expansion and contraction device 40.

When the hip joint torque MYh is smaller than the threshold value -M1 (S27), that is, when a torque acts in the direction of pivoting the hip joint 7 backward (counterclockwise in FIG. 16), if the knee angle θ is smaller than the threshold value θk2 (S30, region AR3 in FIG. 18), the motor M is stopped (S31). Accordingly, it is possible to avoid contact between the mechanical elements inside the expansion and contraction device 40.

When the hip joint torque MYh is larger than a threshold value M1 (S31), that is, when a torque acts in a direction of pivoting the hip joint 7 forward (clockwise in FIG. 16), if the knee angle θ is equal to or smaller than a threshold value θk3 (θk3 > θk2) (S32, region AR2 in FIG. 18), the motor voltage calculated in step S26 is applied (S33). Accordingly, a motor torque and the hip joint torque MYh are balanced, and the knee joint mechanism 30 can be bent. The threshold value θk3 is a critical value for avoiding contact between the mechanical elements inside the expansion and contraction device 40, and is a critical value in a direction opposite to that of step 30.

When the hip joint torque MYh is larger than the threshold value M1 (S31), that is, when a torque acts in the direction of pivoting the hip joint 7 forward (clockwise in FIG. 16), if the knee angle θ is larger than the threshold value θk3 (S34, region AR3 in FIG. 18), the motor M is stopped (S35). Accordingly, it is possible to avoid contact between the mechanical elements inside the expansion and contraction device 40.

When the hip joint torque MYh is equal to or larger than the threshold value -M1 and is equal to or smaller than the threshold value M1 (S36, region AR4 in FIG. 18), the motor M is stopped (S37). At this time, by maintaining engagement of the upper clutch 50U in the first gear shift state even when the motor M is stopped, sliding resistance acts on the knee joint mechanism 30. In the case of the electric prosthetic leg 1 of the first embodiment, in order to maintain engagement of the upper clutch 50U, it is preferable to apply a voltage smaller than that in steps S29 and S33.

According to the second example, in the landing determination (S21), when the behavior of the applied weight FZ2 is in an unstable region, the motor M is stopped without determining whether the electric prosthetic leg 1 is a standing leg or a idling leg (S22), or when the behavior of the hip joint torque MYh is in an unstable region, the motor M is stopped without stretching or bending the knee joint mechanism 30 (S37), so that the motor M can be prevented from being driven in a status not intended by the user.

Although various embodiments have been described above with reference to the drawings, it is needless to say that the present invention is not limited to these examples. It is apparent that those skilled in the art can conceive of various modifications and changes within the scope described in the claims, and it is understood that such modifications and changes naturally fall within the technical scope of the present invention. In addition, respective constituent elements in the above-described embodiments may be freely combined without departing from the gist of the invention.

For example, in the above embodiment, the electric prosthetic leg is exemplified as one embodiment of the joint device of the present invention. However, the present invention is not limited thereto, and may be applied to an upper limb (arm joint), may be an animal other than a human being as a wearing subject, or may be a robot.

In the above embodiment, the power of one motor M is transmitted to the first transmission mechanism T1 and the second transmission mechanism T2 via the connection and disconnection mechanism 50. However, the present invention is not limited thereto, and a connection and disconnection mechanism may be provided between each of two power sources and a respective one of the first transmission mechanism T1 and the second transmission mechanism T2.

Further, both the first transmission mechanism T1 and the second transmission mechanism T2 are not necessarily provided, and only one of the first transmission mechanism T1 and the second transmission mechanism T2 may be provided.

The connection and disconnection mechanism 50 is not limited to the dog clutch, and may be another clutch mechanism such as a friction clutch or a centrifugal clutch, or may be a clutchless mechanism such as a continuous transmission gear ratio switching mechanism.

In the present specification, at least the following matters are described. Corresponding constituent elements and the like in the embodiment described above are shown in parentheses, but the present invention is not limited thereto.

> (1) A joint device (electric prosthetic leg 1) including:
>
>> a first member (lower-knee member 10);
>> a second member (upper-knee member 20);

a coupling portion (knee joint mechanism 30) which couples the first member and the second member so that an angle formed by the first member and the second member is changeable; and
an expansion and contraction device (expansion and contraction device 40) configured to change the angle formed by the first member and the second member by expanding and contracting, in which:

> the expansion and contraction device includes:
>
>> a power source (motor M); and
>> a power transmission portion (transmission T) configured to transmit power of the power source;
>
> the power transmission portion includes:
>
>> a first power transmission path (first transmission mechanism T1) configured to transmit the power at a first transmission gear ratio; and
>> a second power transmission path (second transmission mechanism T2) configured to transmit the power at a second transmission gear ratio different from the first transmission gear ratio;
>
> the expansion and contraction device includes:
>
>> a first connection and disconnection mechanism (upper clutch 50U) configured to switch disconnection and connection of power in the first power transmission path; and
>> a second connection and disconnection mechanism (lower clutch 50D) configured to switch disconnection and connection of power in the second power transmission path;
>
> the joint device further includes:
>
>> a control unit (motor control unit 108, connection and disconnection mechanism control unit 110) which controls the power source, the first connection and disconnection mechanism, and the second connection and disconnection mechanism; and
>> an applied weight acquisition unit (applied and extracted weight acquisition unit 102, thigh applied weight acquisition unit 112) which acquires an applied weight (applied weight FZ, applied weight FZ2) applied to the joint device, and

the control unit controls at least one of the first connection and disconnection mechanism and the second connection and disconnection mechanism based on the applied weight acquired by the applied weight acquisition unit.

According to (1), the coupling portion can be stretched and bent via the power transmission portion which transmits power of the power source. Since the power transmission portion includes two power transmission paths having different transmission gear ratios, an operation speed and generated power of stretching and bending of the coupling portion can be switched.

It is possible to appropriately switch between the two power transmission paths by the first connection and disconnection mechanism and the second connection and disconnection mechanism.

Further, by controlling at least one of the first connection and disconnection mechanism and the second connection and disconnection mechanism based on the applied weight applied to the joint device, the coupling portion can be stretched or bent by power from the drive source.

(2) The joint device according to (1), in which the control unit is configured to switch one of the first connection and disconnection mechanism and the second connection and disconnection mechanism to an engaged state when the applied weight acquisition unit acquires a transition from a state in which no applied weight is applied to a state in which an applied weight is applied.

According to (2), by switching one of the first connection and disconnection mechanism and the second connection and disconnection mechanism to an engaged state at the time of transition, the coupling portion can be stretched or bent smoothly.

(3) The joint device according to (1) or (2), further including:

a control unit (motor control unit 108, connection and disconnection mechanism control unit 110) configured to control the power source, the first connection and disconnection mechanism, and the second connection and disconnection mechanism; and
a traveling direction acquisition unit configured to acquire a traveling direction of a wearing subject of the joint device, in which
the control unit configured to control at least one of the first connection and disconnection mechanism and the second connection and disconnection mechanism based on the traveling direction acquired by the traveling direction acquisition unit.

According to (3), by determining ascending and descending states, it is possible to appropriately determine a status in which the coupling portion is stretched and a status in which the coupling portion is bent.

(4) The joint device according to (3), in which:

when the first transmission gear ratio is a ratio of a post-shift rotation speed to a pre-shift rotation speed which is a rotation speed on a side of the power source in the first power transmission path and the second transmission gear ratio is a ratio of a post-shift rotation speed to a pre-shift rotation speed which is a rotation speed on the side of the power source in the second power transmission path, the first transmission gear ratio is configured to become smaller than the second transmission gear ratio;
the control unit brings the first connection and disconnection mechanism into an engaged state when the control unit acquires that the wearing subject moves upward in a vertical direction.

According to (4), a large amount of power can be generated by bringing the first transmission path having a small transmission gear ratio into the connected state at the time of ascending.

(5) The joint device according to (4), in which:

the second member is configured to be attachable to a first portion (thigh 4) of the wearing subject out of the first portion and a second portion (upper body 3) which pivots relative to the first portion; and
the joint device further includes a pivoting force acquisition unit (hip joint torque calculation unit 104) which acquires a pivoting force (hip joint torque MYh) of the first portion relative to the second portion.

According to (5), by acquiring the pivoting force of the first portion relative to the second portion, it is possible to more appropriately set the amount of power for stretching.

(6) The joint device according to (5), in which the pivoting force acquisition unit acquires the pivoting force based on the angle formed by the first member and second member.

According to (6), the pivoting force can be accurately and easily acquired.

(7) The joint device according to (5) or (6), in which
the pivoting force acquisition unit acquires the pivoting force based on a length of the first portion (length L of thigh 4).

According to (7), the pivoting force can be accurately and easily acquired.

(8) The joint device according to any one of (5) to (7), further including:

an applied weight acquisition unit (applied and extracted weight acquisition unit 102) configured to acquire an applied weight (applied weight FZ) applied to the first member, in which
the pivoting force acquisition unit is configured to acquire the pivoting force based on the applied weight acquired by the applied weight acquisition unit.

According to (8), the pivoting force can be accurately and easily acquired.

(9) The joint device according to any one of (5) to (8), further including:

a control unit (motor control unit 108, connection and disconnection mechanism control unit 110) configured to control the power source, the first connection and disconnection mechanism, and the second connection and disconnection mechanism, in which the control unit is configured to control an amount of power generated from the power source based on the pivoting force acquired by the pivoting force acquisition unit.

According to (9), the amount of power for stretching can be appropriately set.

(10) A joint device (electric prosthetic leg 1) including:

a first member (lower-knee member 10);
a second member (upper-knee member 20);
a coupling portion (knee joint mechanism 30) which couples the first member and the second member so that an angle formed by the first member and the second member is changeable; and
an expansion and contraction device (expansion and contraction device 40) configured to change the angle formed by the first member and the second member by expanding and contracting, in which:

the expansion and contraction device includes:

a power source (motor M); and
a power transmission portion (transmission T) configured to transmit power of the power source;

the second member is configured to be attachable to the first portion (thigh 4) of a wearing subject of the joint device out of the first portion and a second portion (upper body 3) which pivots relative to the first portion;
the joint device further includes:

a control unit (motor control unit 108) configured to control the power source; and
a pivoting force acquisition unit (hip joint torque calculation unit 104) configured to acquire a pivoting force (hip joint torque MYh) of the first portion relative to the second portion; and
the control unit is configured to control an amount of power generated from the power source based on the pivoting force acquired by the pivoting force acquisition unit.

According to (10), the amount of power for stretching can be appropriately set.

(11) A joint device (electric prosthetic leg 1) including:

a first member (lower-knee member 10);
a second member (upper-knee member 20);
a coupling portion (knee joint mechanism 30) which couples the first member and the second member so that an angle formed by the first member and the second member is changeable; and
an expansion and contraction device (expansion and contraction device 40) configured to change the angle formed by the first member and the second member by expanding and contracting, in which:

the expansion and contraction device includes:

a power source (motor M); and
a power transmission portion (transmission T) configured to transmit power of the power source;

the power transmission portion includes:

a first power transmission path (first transmission mechanism T1) configured to transmit the power at a first transmission gear ratio; and
a second power transmission path (second transmission mechanism T2) configured to transmit the power at a second transmission gear ratio different from the first transmission gear ratio;

the expansion and contraction device includes:

a first connection and disconnection mechanism (upper clutch 50U) configured to switch disconnection and connection of power in the first power transmission path; and
a second connection and disconnection mechanism (lower clutch 50D) configured to switch disconnection and connection of power in the second power transmission path;

the joint device further includes:

a control unit (motor control unit 108, connection and disconnection mechanism control unit 110) configured to control the power source, the first connection and disconnection mechanism, and the second connection and disconnection mechanism; and

a traveling direction acquisition unit configured to acquire a traveling direction of a wearing subject of the joint device; and

the control unit is configured to control at least one of the first connection and disconnection mechanism and the second connection and disconnection mechanism based on the traveling direction acquired by the traveling direction acquisition unit.

According to (11), by determining ascending and descending states, it is possible to appropriately determine a status in which the coupling portion is stretched and a status in which the coupling portion is bent.

The present application is based on the Japanese patent application (Japanese Patent Application No. 2021-032992) filed on March 2,2021, and the contents thereof are incorporated herein by reference.

REFERENCE SIGNS LIST

1: electric prosthetic leg (joint device)
3: upper body (second portion)
4: thigh (first portion)
10: lower-knee member (first member)
20: upper-knee member (second member)
30: knee joint mechanism (coupling portion)
40: expansion and contraction device
50D: lower clutch (second connection and disconnection mechanism)
50U: upper clutch (first connection and disconnection mechanism)
102: applied and extracted weight acquisition unit (applied weight acquisition unit)
104: hip joint torque calculation unit (pivoting force acquisition unit)
108: motor control unit (control unit)
110: connection and disconnection mechanism control unit (control unit)
112: thigh applied weight acquisition unit (applied weight acquisition unit)
M: motor (power source)
T: transmission (power transmission portion)
T1: first transmission mechanism (first power transmission path)
T2: second transmission mechanism (second power transmission path)

**Claims**

1. A joint device comprising:

a first member;

a second member;
a coupling portion which couples the first member and the second member so that an angle formed by the first member and the second member is changeable; and
an expansion and contraction device configured to change the angle formed by the first member and the second member by expanding and contracting, wherein:

the expansion and contraction device includes:

a power source; and
a power transmission portion configured to transmit power of the power source;

the power transmission portion includes:

a first power transmission path configured to transmit the power at a first transmission gear ratio; and
a second power transmission path configured to transmit the power at a second transmission gear ratio different from the first transmission gear ratio;

the expansion and contraction device includes:

a first connection and disconnection mechanism configured to switch disconnection and connection of power in the first power transmission path; and
a second connection and disconnection mechanism configured to switch disconnection and connection of power in the second power transmission path; and

the joint device further comprises:

a control unit configured to control the power source, the first connection and disconnection mechanism, and the second connection and disconnection mechanism; and
an applied weight acquisition unit which acquires an applied weight applied to the joint device, and
the control unit controls at least one of the first connection and disconnection mechanism and the second connection and disconnection mechanism based on the applied weight acquired by the applied weight acquisition unit.

2.  The joint device according to claim 1, wherein the control unit is configured to switch one of the first connection and disconnection mechanism and the second connection and disconnection mechanism to an engaged state when the applied weight acquisition unit acquires a transition from a state in which no applied weight is applied from an outside to a state in which an applied weight is applied from the outside.

3.  The joint device according to claim 1 or 2, further comprising:

    a control unit configured to control the power source, the first connection and disconnection mechanism, and the second connection and disconnection mechanism; and
    a traveling direction acquisition unit configured to acquire a traveling direction of a wearing subject of the joint device, wherein
    the control unit is configured to control at least one of the first connection and disconnection mechanism and the second connection and disconnection mechanism based on the traveling direction acquired by the traveling direction acquisition unit.

4.  The joint device according to claim 3, wherein:

    when the first transmission gear ratio is a ratio of a post-shift rotation speed to a pre-shift rotation speed which is a rotation speed on a side of the power source in the first power transmission path and the second transmission gear ratio is a ratio of a post-shift rotation speed to a pre-shift rotation speed which is a rotation speed on the side of the power source in the second power transmission path, the first transmission gear ratio is configured to become smaller than the second transmission gear ratio;
    the control unit brings the first connection and disconnection mechanism into an engaged state when the control unit acquires that the wearing subject moves upward in a vertical direction.

5.  The joint device according to claim 4, wherein:

    the second member is configured to be attachable to a first portion of the wearing subject out of the first portion and a second portion which pivots relative to the first portion; and
    the joint device further includes a pivoting force acquisition unit which acquires a pivoting force of the first portion relative to the second portion.

6.  The joint device according to claim 5, wherein the pivoting force acquisition unit acquires the pivoting force based on the angle formed by the first member and the second member.

7.  The joint device according to claim 5 or 6, wherein the pivoting force acquisition unit acquires the pivoting force based on a length of the first portion.

8.  The joint device according to any one of claims 5 to 7, further comprising:

    an applied weight acquisition unit configured to acquire an applied weight applied to the first member, wherein
    the pivoting force acquisition unit is configured to acquire the pivoting force based on the applied weight acquired by the applied weight acquisition unit.

9.  The joint device according to any one of claims 5 to 8, further comprising:

    a control unit configured to control the power source, the first connection and disconnection mechanism, and the second connection and disconnection mechanism, wherein
    the control unit is configured to control an amount of power generated from the power source based on the pivoting force acquired by the pivoting force acquisition unit.

10. A joint device comprising:

    a first member;
    a second member;
    a coupling portion which couples the first member and the second member so that an angle formed by the first member and the second member is changeable; and
    an expansion and contraction device configured to change the angle formed by the first member and the second member by expanding and contracting, wherein:

        the expansion and contraction device includes:

            a power source; and
            a power transmission portion configured to transmit power of the power source;

        the second member is configured to be attachable to the first portion of a wearing subject of the joint device out of the first portion and a second portion which pivots relative to the first portion;
        the joint device further comprises:

a control unit configured to control the power source; and

a pivoting force acquisition unit configured to acquire a pivoting force of the first portion relative to the second portion; and

the control unit is configured to control an amount of power generated from the power source based on the pivoting force acquired by the pivoting force acquisition unit.

**11.** A joint device comprising:

a first member;

a second member;

a coupling portion which couples the first member and the second member so that an angle formed by the first member and the second member is changeable; and

an expansion and contraction device configured to change the angle formed by the first member and the second member by expanding and contracting, wherein

the expansion and contraction device includes:

a power source; and

a power transmission portion configured to transmit power of the power source;

the power transmission portion includes:

a first power transmission path configured to transmit the power at a first transmission gear ratio; and

a second power transmission path configured to transmit the power at a second transmission gear ratio different from the first transmission gear ratio;

the expansion and contraction device includes:

a first connection and disconnection mechanism configured to switch disconnection and connection of power in the first power transmission path; and

a second connection and disconnection mechanism configured to switch disconnection and connection of power in the second power transmission path;

the joint device further comprises:

a control unit configured to control the power source, the first connection and disconnection mechanism, and the second connection and disconnection mechanism; and

a traveling direction acquisition unit configured to acquire a traveling direction of a wearing subject of the joint device; and

the control unit is configured to control at least one of the first connection and disconnection mechanism and the second connection and disconnection mechanism based on the traveling direction acquired by the traveling direction acquisition unit.

## FIG. 1

FIG. 2

# FIG. 3

*FIG. 4*

*FIG. 5A*

*FIG. 5B*

FIG. 5C

FIG. 6

*FIG. 7*

*FIG. 8*

FIG. 9

FIG. 10

100

201
APPLIED AND EXTRACTED WEIGHT SENSOR

APPLIED AND EXTRACTED WEIGHT ACQUISITION UNIT ~102

HIP JOINT TORQUE CALCULATION UNIT ~104

203
SHIN IMU

THIGH ANGLE CALCULATION UNIT ~106

205
KNEE ANGLE SENSOR

KNEE ANGLE CALCULATION UNIT ~103

MOTOR CONTROL UNIT ~108

INTERMITTENT MECHANISM CONTROL UNIT ~110

# FIG. 11

FIG. 12

EP 4 303 462 A1

## FIG. 13

# FIG. 14

## FIG. 15

100

201 — APPLIED AND EXTRACTED WEIGHT SENSOR → APPLIED AND EXTRACTED WEIGHT ACQUISITION UNIT — 102

203 — SHIN IMU → THIGH ANGLE CALCULATION UNIT — 106

205 — KNEE ANGLE SENSOR → KNEE ANGLE CALCULATION UNIT — 103

207 — KNEE IMU

HIP JOINT TORQUE CALCULATION UNIT — 104

THIGH APPLIED WEIGHT ACQUISITION UNIT — 112

KNEE TORQUE ACQUISITION UNIT — 113

KNEE SHEAR FORCE ACQUISITION UNIT — 114

MOTOR CONTROL UNIT — 108

INTERMITTENT MECHANISM CONTROL UNIT — 110

# FIG. 16

## FIG. 17

*FIG. 18*

# FIG. 19

FZ2(N)

POSITIVE

T12

0

TIME (s)

-T12

FZ2

NEGATIVE

MYh(N·m)

POSITIVE

0

TIME (s)

MYh

NEGATIVE

## INTERNATIONAL SEARCH REPORT

| International application No. |
| --- |
| **PCT/JP2022/007967** |

**A.    CLASSIFICATION OF SUBJECT MATTER**

*F16D 43/202*(2006.01)i; *A61F 2/62*(2006.01)i; *F16H 21/10*(2006.01)i; *F16H 25/20*(2006.01)i; *F16H 35/00*(2006.01)i; *F16H 37/12*(2006.01)i

FI:    F16H21/10 Z; A61F2/62; F16H37/12 Z; F16H25/20 Z; F16H35/00 B; F16D43/202

According to International Patent Classification (IPC) or to both national classification and IPC

**B.    FIELDS SEARCHED**

Minimum documentation searched (classification system followed by classification symbols)

F16D43/202; A61F2/62; F16H21/10; F16H25/20; F16H35/00; F16H37/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| X | WO 2018/092325 A1 (CYBERDYNE INC) 24 May 2018 (2018-05-24) | 10 |
|   | paragraphs [0025]-[0034], [0040], [0113]-[0116], fig. 1-13 |   |
| A |   | 1-9, 11 |
| A | WO 2020/203762 A1 (HONDA MOTOR CO LTD) 08 October 2020 (2020-10-08) | 1-11 |
| A | JP 5-146978 A (HONDA MOTOR CO LTD) 15 June 1993 (1993-06-15) | 1-11 |
| A | JP 2013-136136 A (MORIKAWA, Atsuo) 11 July 2013 (2013-07-11) | 1-11 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **28 April 2022** | **17 May 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2022/007967**

| Patent document cited in search report | | | Publication date (day/month/year) | Patent family member(s) | Publication date (day/month/year) |
|---|---|---|---|---|---|
| WO | 2018/092325 | A1 | 24 May 2018 | US 2019/0328553 A1 paragraphs [0038]-[0047], [0053], [0131]-[0134], fig. 1-13<br>EP 3542762 A1 | |
| WO | 2020/203762 | A1 | 08 October 2020 | (Family: none) | |
| JP | 5-146978 | A | 15 June 1993 | (Family: none) | |
| JP | 2013-136136 | A | 11 July 2013 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H1119105 A

- JP 2021032992 A